# EUROPEAN PATENT APPLICATION

(11) **EP 1 788 080 A1**
(43) Date of publication of application: **23.05.2007**
(21) Application number: 05025463.0
(22) Date of filing: 22.11.2005
(51) Int. Cl.: C12N 9/20, C12N 15/55, C12P 7/64, C11B 3/00

(54) **Use of a thermostable phospholipase in the degumming of an oil or fat, and a method for obtaining a thermostable phopholipase**

(71) Applicant: SÜD-CHEMIE AG, 80333 München (DE)
(72) Inventor: Jackisch, Björn-Oliver, Dr., 30916 Isernhagen (DE); Simmler-Hübenthal, Hubert, 85368 Moosburg (DE); Zschau, Werner, Dr., 82237 Wörthsee-Steinebach (DE); Bornscheuer, Uwe, Prof. Dr., 17487 Greifswald (DE); Durban, Markus, 17487 Greifswald (DE); Riemer, Christoph, Dr., 81673 München (DE)
(74) Representative: Westendorp, Michael Oliver

(57) **Abstract**

The invention relates to the use of a phospholipase comprising SEQ ID N0 : 1 (TNPEDWIHG) or having at least 75% sequence identity to SEQ ID N0 : 1 and comprising aspartate at position 5 of SEQ ID N0 : 1 in a process for the cleavage and/or removal of at least one substrate of the phospholipase from a composition comprising the substrate of the phospholipase and at least one oil or fat. Further provided is a method of obtaining the phospholipase or for increasing the thermostability of a phospholipase not comprising SEQ ID NO:1.

## Description

The present invention relates to the use of a phospholipase, especially at elevated temperatures, in particular its preferred use in the degumming of edible oil or fat, and a method of obtaining the phospholipase.

### Field of the Invention

Phospholipases are enzymes that hydrolyze ester bonds of phospholipids.

Several types of phospholipases are known which differ in their specificity according to the position of the bond attacked in the phospholipid molecule.

Phospholipase A1 (PLA1) removes the 1-position fatty acid to produce free fatty acid and1-lyso-2-acylphospholipid.

Phospholipase A2 (PLA2) removes the 2-position fatty acid to produce free fatty acid and 1-acyl-2-lysophospholipid. PLA1 and PLA2 enzymes can be intra- or extracellular, membrane-bound or soluble. Phospholipase B (also termed as lysophospholipase) (EC classification as EC 3.1.1.5) can hydrolyse the remaining glycerol-fatty acid ester bond in the lysophospholipid.

Phospholipase C (PLC, EC classification as 3.1.4.3) cleaves phospholipids stereospecifically into 1,2-diacylglycerides and phosphate monoesters. Some PLCs are more substrate specific than others. The inositol-specfic PLC [E.C.3.1.4.10] for example cleaves only phosphatidylinositol-4,5-bisphosphate into 1,2-diacylglycerides and inositol-(1,4,5)-triphosphate, which is an important second messenger in the calcium metabolism. In contrast, the phosphatidyichoiine-specific phospholipase C (PC-PLC) [E.C.3.1.4.3] prefers phosphatidylcholine. Due to its high structural and catalytical similarity to mammalian PLCs the enzyme from B. cereus is also subject of medical research. Moreover, this enzyme is used in basic research in the areas of lipid metabolism, chemistry of lipids, blood coagulation or of eucaryotic cell membranes. The physiological function of the PC-PLC is not known but the enzyme seems to be part of a phosphate retrieval system.

The PC-PLC of B. cereus is an extracellular, monomeric tri-zinc protein (Little, C. and A.-B. Otnaess, The metal ion dependence of phospholipase C from Bacillus cereus. Biochim. Biophys. Acta, 1975. 391: p. 326-333). It is synthesized as a 283 amino acid long pre-proenzyme from which first the 24 aa long presequence and the the 14 aa long prosequence is cleaved. The mature enzyme has a length of 245 aa and a calculated molecular weight of 28520 Da, including the three zinc ions. On the SDS-PAGE gels a band can be seen at about 27 kDa.

The PC-PLC gene has a length of 852 nucleotides and it is located on the bacterial chromosome (Johansen, T., et al., Cloning and sequencing of the gene encoding the phosphatidylcholine-preferring phospholipase C of Bacillus cereus. Gene, 1988. 65: p. 293-304).

The PC-PLC can also be used for the synthesis of optically pure 1,2-diglycerides. With these diglycerides, synthetic phospholipids can be synthesized which are interesting for the pharmaceutical industry.

Phospholipase D (EC classification as EC 3.1.4.4.) hydrolyses the phosphoric acid diester bond to yield a phosphodiglyceride (phosphatidic acid) and organic compound as cleavage products.

Natural fat and natural oil are solid or liquid mixtures mainly comprised of glycerides. Depending on whether they are solid or liquid at room temperature they are specified as fat or oil, respectively.

Crude fat or crude oil contains regularly accompanying components such as hydratable and non-hydratable phosphatides (phospholipids). Hydratable phosphatides can be removed from crude fat or oil by treating said crude fat or oil with water in a so-called wet or water degumming process. Typically, the amount of water is 75% of the phosphatides content, which is typically 1 to 1.5 %. The temperature is not highly critical, although separation of the hydrated gums is better if the viscosity of the oil is reduced at 50 to 80 °C. However, said non-hydratable phosphatides remain within the crude fat or oil preparation and still cause gumminess. Therefore, the remaining non-hydratable phosphatides have to be removed in an additional degumming process. In this respect, phospholipases may be used.

In the past, phospholipases A and B have been primarily used in degumming processes for refining crude fat and oil. An example is the Enzymax® process for degumming of edible oil by Lurgi in which the fraction of non-hydratable glycerophospholipids is removed by enzymatic hydrolysis.

In a current process, a phospholipase of the A or B types, e.g., phospholipase A2 derived from pancreas gland is employed.

WO 98/26057 discloses a method for reducing the content of phosphorus components in edible oil using a phospholipase A1 or phospholipase A2. Phospholipase C (PLC) also has been considered for oil degumming, see WO 03/089620 A2.

DE 195 27 274 A1 is directed to an enzymatic process for degumming vegetable oil using a phospholipase displaying phospholipase A1 and/or phospholipase A2 activity as well as lysophospholipase activity. A phospholipase isolated from Aspergillus has been used in this process.

EP 0 622 446 relates to a process for refining oil and fat using an enzyme having activity to decompose glycerol-fatty acid ester bonds in glycerophospholipids.

Common to all phospholipases of the A and B type used in the aforementioned state of art is the inevitable release of additional free fatty acids which have to be removed or neutralised during further refining of the fat and oil, and the formation of lysophosphatides which may act as emulsifiers and thus interfere with phase separation.

High phosphatide oils such as soy, canola and sunflower oil are processed differently than other oils such as palm. Unlike the steam or "physical refining" process for low phosphatide oils, these high phosphorous oils require special chemical and mechanical treatments to remove the phosphorous-containing phospholipids. These oils are typically refined chemically in a process that entails neutralizing the free fatty acids to form soap and an insoluble gum fraction. The neutralization process is highly effective in removing free fatty acids and phospholipids but this process also results in significant yield losses and sacrifices in quality. In some cases, the high phosphatide crude oil is degummed in a step preceding caustic neutralization. This is the case for soy oil utilized for lecithin wherein the oil is first water or acid degummed.

Thus, there is a constant need for improved phospholipases (PLs) with properties particularly suitable for their industrial application e.g. in the degumming of edible oils, in particular at elevated temperatures.

It was therefore one object of the present invention to provide phospholipases which are particularly suitable for industrial application e.g. in the degumming of oil or fat, in particular at elevated temperatures and to avoid the disadvantages of PLs known from prior art.

According to a first aspect of the present invention the phospholipase used comprises a polypeptide comprising SEQ ID NO : 1:
TNPEDWIHG
or an amino acid sequence having at least 75% sequence identity or being substantially identical to SEQ ID NO : 1, and comprising aspartate at position 5 of SEQ ID NO : 1. The standard one-letter abbreviations of amino acids are used (i.e. T=threonine (Thr); N=asparagine (Asn), P=proline (Pro); E=glutamate (glutamic acid; Glu); D=aspartate (aspartic acid; Asp); W=tryptophan (Trp); I=isoleucine (Ile); H=histidine (His); G=glycine (Gly)).

Thus, it was unexpectedly found that the presence of aspartate at the above position remarkably increases the thermostability of a phospholipase. For example, the wildtype PC-PLC of *B. cereus* (see Johansen, T., et al., Cloning and sequencing of the gene encoding the phosphatidylcholine-preferring phospholipase C of Bacillus cereus. Gene, 1988. 65: p. 293-304) comprises glutamate at position 174 of the amino acid sequence (corresponding to position 5 of SEQ ID NO:1). However, an amino acid sequence in which the glutamate at position 174 is exchanged for aspartate shows a much higher thermostability, i.e. a higher phospholipase activity at elevated temperatures.

It was thus surprising that a phospholipase having an aspartate residue in position 5 of SEQ ID NO:1 has a significant influence on the thermostability of the polypeptide comprising this sequence.

Even more surprisingly, it was found that the phospholipases as described above and as will be described in further detail below have a superior activtity and thermal stability regarding the cleavage of phospholipids in an oil or fat containing composition such as a composition containing a plant or animal oil or fat.

Thus, one aspect of the present invention is directed to the use of a phospholipase as described herein for the cleavage and/or removal of at least one substrate of the phospholipase from a composition comprising the substrate of the phospholipase and at least one oil or fat.

According to a preferred aspect, the substrate of the phospholipase comprises or consists of at least one phospholipid. Preferably, the phospholipase is a phospholipase C (PLC). According to a further preferred aspect, the phospholipase is isolated or originates from B. cereus, B. thuringiensis, B. subtilis or B. anthracis. The substrates cleaved by PLC, including PC-PLC, have already been described above. Preferably, the substrate of the phospholipase comprises or consists of at least one non-hydratable phospholipid as described above.

According to a further preferred aspect of the present invention, the process in which the phospholipase is used is a process comprising the following steps:
a) providing the phospholipase,
b) providing a composition comprising at least one substrate for the phospholipase and at least one oil or fat;
c) contacting the phospholipase with the composition to obtain a mixture comprising the phospholipase and its substrate (and at least one oil or fat);
d) incubating the mixture, preferably at a temperature above 20 °C.

In the incubation, the phospholipase is allowed to cleave the substrate. Preferably, the cleaved substrate is then removed from the oil or fat.

It is further preferred that the process is a process for the treatment or degumming of an oil or fat, in particular a plant oil, an animal oil, an algae or a fish oil or a fat. Non-limiting examples of preferred plant oils are a soybean oil, a rapeseed oil, a corn oil, an oil from a palm kernel, a canola oil, a sunflower oil, a sesame oil or a peanut oil.

The conditions for the incubation are chosen such that the phospholipase can catalyze the cleavage of a phospholipid in the composition.

The composition is preferably a liquid composition, i.e. not a solid composition. It preferably comprises an oil phase and a water phase. Thus, according to a preferred aspect, the composition is in the form of an emulsion of the oil phase and a water phase.

According to a further aspect of the invention, for achieving improvements in the overall oil refining process exceeding the degumming step, the phospholipase described herein as applied in the degumming can beneficially be combined with other hydrolyzing enzymes such as at least one of phospholipases of the types A, B, D and phosphatases as well as with proteases, cellulases, hemicellulases, pectinases, xy-lanases to beneficially influence hydrolysis equilibria and to simultaneously remove other oil-seed derived impurities such as residual protein as well as oligo- or poly-saccharide components. However, according to one aspect of the invention, the phospholipase C as described herein is used in the degumming process in the absence on other phospholipases, in particular phospholipases of the types A, B and D, and also of phosphatases.

Methods of enzymatic degumming of oils or fat and the general parameters and conditions to be used are known in the art, for example from [K. Dahlke, Chem. Eng. Technol. 1998; H. Buchold, Fat. Sci. Technol. 1993], DE 195 27 274 A and EP 0 622 446 A herein incorporated by reference.

According to a further preferred aspect, the degumming (treatment) using the phospholipase as decribed herein is performed using a water degummed oil or fat (see above). For example, the water-degumming of a crude oil or fat may be achieved by thoroughly mixing hot water and warm oil or fat having a temperature of between 60°C to 90°C for 30 to 60 minutes. Also, an acid treatment may be performed before the enzymatic degumming, in particular a treatment using citric acid as known in the art. As an example only, 100 g of a water-degummed rapeseed oil may be treated at a temperature preferably in the range from 30 to 80°C, e.g. at 60°C, with 2g of a 12.5% solution of citric acid in water for about 15 min. The acid treatment is preferably followed by a neutralisation step to adjust the pH between about 5 and 6.5, preferably using NaOH. Preferably, the phospholipase as described herein is then added.

Preferably the amount of phospholipase added is between 10 µg and 5 g/kg of oil or fat to be treated. Also, preferably between 10 and 500.000 units of the phospholipase are used per kg of oil or fat to be treated. In most cases, the incubation with the phospholipase will be between 5 minutes and 24 hours, more preferable between 15 min and 10 hours. Preferably the mixture is agitated during the incubation with the phospholipase. This may be achieved by any known agitation method, including shaking, stirring or sonification.

The oil and water phases, respectively, may then preferably be separated, e.g. by centrifugation. The oil phase shows a decreased P-content as indicated by standard IPC analysis. The samples were prepared according to AOCS Official Method Ca 15-75 and measurement was made in kerosene and otherwise in accordance with DIN 38406T22. (see also Nölte J. "ICP Emissionsspektrometrie für Praktiker", Wiley-VCH; 2002). Alternatively, the P-content may be measured as indicated in WO98/26057, pages 75/76. As an example only, typical P-contents of oils are as follows: Water degummed rape seed or soybean oil:100 - 300 ppm P; After citric acid treatment: (15 min., 60°C): 30 - 50 ppm P; After treatment with phospholipase: < 20 ppm P.

It is also possible to perform the degumming step using the phospholipase as described herein on a crude oil or fat, i.e. an oil or fat not previously water degummed or acid treated. Also, for example if the water degummed oil or fat already has a P-content of less than about 50 ppm it is possible to eliminate the acid treatment step and to directly proceed with the phosholipase incubation (enzymatic degumming).

According to one preferred aspect, the phospholipase is added in the form of a solution, preferably an aqueous solution. The aqueous solution may also comprise auxiliary components selected from the group consisting of buffer agents, inorganic salts, solvents, inert solids and mixtures thereof. Appropriate buffer systems, e.g., are made from aqueous solutions of salts or organic acids, amino acids, phosphate, amines or ammonia in concentrations between 0,01 M and 1 M at pH 2 to 10. Preferably, alkali metal salts of citric acid, acetic acid, glycine and/or hydrochlorides of tris(hydroxymethyl)amine and ammonia at 0,1 M to 0,2 M at pH 4 to 8 are used. Preferably, the phospholipase is solved in an aqueous buffer solution such as glycine buffer, citric acid buffer, etc.. Citrate containing buffers have been found to be very suitable, in particular 1 to 15 mM citrate buffers at pH 4.5 to 6.5. According to a further preferred aspect of the invention it has also been found that borax (borate) containing buffers are particularily suitable for the phospholipase as described herein and allow high activities in the process as described above. Thus suitable borax (borate) buffers comprise 20 to 60 mM borax (borate) at pH 6 to 8.

The phospholipases as described herein are capable of reducing the phosphorus level (P-content) of an oil or fat, e.g. a water degummed oil or fat, from 200 ppm or more to preferably less than 20 ppm, preferably less than 10 ppm, particularly preferred less than 5 ppm. Apart from the use of the phospholipase in solid e.g. power or granular form and the use as a solution as described above, the phospholipase may also be immobilized on a carrier or carrier particles. This allows rapid and easy separation of the enzyme from the enzymatically degummed oil or fat and from the aqueous solution. This separation may be performed e.g. by centrifuging, filtering or settling. The thermostable enzyme is immobilised on said carrier particles by adsorption or chemical bonding while conserving its enzymatic activity. Suitable methods and coupling agents are known to the skilled person. For example, reactive N-hydroxy succinimide ester groups on carrier particles which react with amino groups of the enzymes may be used. Also, BrCN-activated Sepharose®-particles can be employed. Magnetic carrier particles may further facilitate separation of the immobilised phospholipase after enzymatic degumming of the oil or fat.

According to one embodiment, the phospholipase is bound to a filter and the phospholipid-containing fat or oil is passed through the filter, or the polypeptide is added to a solution comprising the phospholipid-containing fat or oil and then the solution is passed through a filter.

According to one aspect, the invention also provides a process for degumming vegetable and/or animal oil or fat, wherein the process comprises the steps of contacting said vegetable and/or animal oil or fat with an aqueous solution of a phospholipase as described herein at a temperature of between 20°C and 95°C for a period of time sufficient to reduce the amount of phospholipids in said vegetable and/or animal oil or fat; optionally separating said aqueous solution of a thermostable enzyme from the vegetable and/or animal oil or fat.

As described above, it has been surprisingly found that a phospholipase, in particular a PLC, having an aspartate residue in position 5 of SEQ ID NO:1 has a significantly improved thermostability or thermotolerance. A phospholipase as described herein therefore allows to perform processes using this enzyme at high temperatures and surprisingly high phospholipids-cleavage activity.

Therefore, according to a further preferred aspect of the present invention, the temperature used in the incubation (step d) of the process described above) is 37 °C or above, preferably 50 °C or above, preferably 55 °C or above, preferably 60 °C or above, more preferred 65 °C or above, further preferred 75 °C or above, in particular 80 °C or above or even 85 °C or above. Particularly preferred said temperature is in the range of between 60°C to 70°C.

Further aspects of the invention are described below. The terms and expressions used herein shall have the following meaning.

### Definitions

The meaning of the following terms and expressions is known to the skilled person and is e.g. evident from WO 03/089620 A2, the respective disclosure of which is hereby incorporated by reference. In summary:

The term "phospholipase" is meant to encompass enzymes having any phospholipase activity, for example, cleaving a glycerolphosphate ester linkage (catalyzing hydrolysis of a glycerolphosphate ester linkage), e. g. , in an oil, such as a vegetable oil. The phospholipase activity of the invention can generate a water extractable phosphorylated base and a diglyceride. The phospholipase activity of the invention also includes hydrolysis of glycerolphosphate ester linkages at high temperatures, low temperatures, alkaline pHs and at acidic pHs. The term "a phospholipase activity" also includes cleaving a glycerolphosphate ester to generate a water extractable phosphorylated base and a diglyceride. The term "a phospholipase activity" also includes cutting ester bonds of glycerin and phosphoric acid in phospholipids.

A "phospholipase C" (PLC) is an enzyme (EC classification as 3.1.4.3) which is capable of cleaving phospholipids into 1,2-diacylglycerides and phosphate monoesters. This includes e.g. inositol-specific PLCs [E.C.3.1.4.10] and phosphatidylcholine-specific PLCs (PC-PLC) [E.C.3.1.4.3]. The NPPC assay as decribed in item 3 of the Examples section may be used to evaluate PLC activity of a polypeptide.

A "coding sequence of" or a "sequence encoding" a particular polypeptide or protein, is a nucleic acid sequence which is transcribed and translated into a polypeptide or protein when placed under the control of appropriate regulatory sequences.

The term "expression cassette" as used herein refers to a nucleotide sequence which is capable of affecting expression of a structural gene (i. e. a protein coding sequence, such as a phospholipase sequence) in a host compatible with such sequences. Expression cassettes include at least a promoter operably linked with the polypeptide coding sequence; and, optionally, with other sequences, e. g., transcription termination signals. Additional factors necessary or helpful in effecting expression may also be used, e. g., enhancers.

"Operably linked" as used herein refers to linkage of a promoter upstream from a DNA sequence such that the promoter mediates transcription of the DNA sequence. Thus, expression cassettes also include plasmids, expression vectors, recombinant viruses, any form of recombinant "naked DNA" vector, and the like.

A "vector" as used herein comprises a nucleic acid which can infect, transfect, transiently or permanently transduce a cell. A vector can be a naked nucleic acid, or a nucleic acid complexed with protein or lipid. The vector optionally comprises viral or bacterial nucleic acids and/or proteins, and/or membranes (e. g., a cell membrane, a viral lipid envelope, etc.). Vectors include, but are not limited to replicons (e. g., RNA replicons, bacteriophages) to which fragments of DNA may be attached and become replicated. Vectors thus include, but are not limited to RNA, autonomous self-replicating circular or linear DNA or RNA (e. g. , plasmids, viruses, and the like, see, e. g. , U. S. Patent No. 5,217, 879), and includes both the expression and non-expression plasmids. If a recombinant microorganism or cell culture is hosting an "expression vector" as used herein, this includes both the possibility of extra-chromosomal circular and linear DNA and DNA that has been incorporated into the host chromosome(s). Where a vector is being maintained by a host cell, the vector may either be stably replicated by the cells during mitosis as an autonomous structure, or is incorporated within the host's genome.

"Plasmids" are commonly designated by a lower case "p" preceded and/or followed by capital letters and/or numbers. The starting plasmids herein are either commercially available, publicly available on an unrestricted basis, or can be constructed from available plasmids in accordance with published procedures. In addition, equivalent plasmids to those described herein are known in the art and will be apparent to the skilled artisan.

The term "gene" means the segment of DNA involved in producing a polypeptide chain, including, inter alia, regions preceding and following the coding region, such as leader and trailer, promoters and enhancers, as well as, where applicable, intervening sequences (introns) between individual coding segments (exons).

The phrases "nucleic acid" or "nucleic acid sequence" as used herein refer to an oligonucleotide, nucleotide, polynucleotide, or to a fragment of any of these, to DNA or RNA (e. g., mRNA, rRNA, tRNA, iRNA) of genomic or synthetic origin which may be single-stranded or double-stranded and may represent a sense or antisense strand, to peptide nucleic acid (PNA), or to any DNA-like or RNA-like material, natural or synthetic in origin, including, e. g., iRNA, ribonucleoproteins (e.g., double stranded iRNAs, e. g. , iRNPs). The term encompasses nucleic acids, i. e. oligonucleotides, containing known analogues of natural nucleotides. The term also encompasses nucleic-acid-like structures with synthetic backbones, see e. g. , Mata (1997) Toxicol. Appl. Pharmacol. 144: 189-197; Strauss-Soukup (1997) Biochemistry 36: 8692-8698; Samstag (1996) Antisense Nucleic Acid Drug Dev. 6 : 153-156.

"Amino acid" or "amino acid sequence" as used herein refer to an oligopeptide, peptide, polypeptide, or protein sequence, or to a fragment, portion, or subunit of any of these, and to naturally occurring or synthetic molecules.

The terms "polypeptide" and "protein" as used herein, refer to amino acids joined to each other by peptide bonds or modified peptide bonds, i. e. peptide isosteres, and may contain modified amino acids other than the 20 gene-encoded amino acids. The term "polypeptide" also includes peptides and polypeptide fragments, motifs and the like. The term also includes glycosylated polypeptides. The peptides and polypeptides of the invention also include all "mimetic" and "peptidomimetic" forms, as described in further detail, below.

As used herein, the term "isolated" means that the material is removed from its original environment (e. g., the natural environment if it is naturally occurring). For example, a naturally occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment. As used herein, an isolated material or composition can also be a "purified" composition, i. e. , it does not require absolute purity; rather, it is intended as a relative definition. Individual nucleic acids obtained from a library can be conventionally purified to electrophoretic homogeneity. In alternative aspects, the invention provides nucleic acids which have been purified from genomic DNA or from other sequences in a library or other environment by at least one, two, three, four, five or more orders of magnitude.

As used herein, the term "recombinant" means that the nucleic acid is adjacent to a "backbone" nucleic acid to which it is not adjacent in its natural environment. In one aspect, nucleic acids represent 5% or more of the number of nucleic acid inserts in a population of nucleic acid "backbone molecules". "Backbone molecules" according to the invention include nucleic acids such as expression vectors, self-replicating nucleic acids, viruses, integrating nucleic acids, and other vectors or nucleic acids used to maintain or manipulate a nucleic acid insert of interest. In one aspect, the enriched nucleic acids represent 15%, 20%, 30%, 40%, 50%, 60%, 70%, 80%, 90% or more of the number of nucleic acid inserts in the population of recombinant backbone molecules. "Recombinant" polypeptides or proteins refer to polypeptides or proteins produced by recombinant DNA techniques; e. g. , produced from cells transformed by an exogenous DNA construct encoding the desired polypeptide or protein. "Synthetic" polypeptides or proteins are those prepared by standard chemical synthesis as known to the skilled person.

A promoter sequence is "operably linked to" a coding sequence when RNA polymerase which initiates transcription at the promoter will transcribe the coding sequence into mRNA, as discussed further, below.

"Oligonucleotide" refers to either a single stranded polydeoxynucleotide or two complementary polydeoxynucleotide strands which may be chemically synthesized. Such synthetic oligonucleotides have no 5'phosphate and thus will not ligate to another oligonucleotide without adding a phosphate with an ATP in the presence of a kinase. A synthetic oligonucleotide will ligate to a fragment that has not been dephosphorylated.

The phrase "substantially identical" in the context of two nucleic acids or polypeptides, refers to two or more sequences that have at least 50%, 60%, 70%, 75%, 80%, 85%, 90%, 95%, 98% or 99% nucleotide or amino acid residue (sequence) identity, when compared and aligned for maximum correspondence, as measured using any one known sequence comparison algorithm, as discussed in detail below, or by visual inspection. In alternative aspects, the invention provides nucleic acid and polypeptide sequences having substantial identity to an exemplary sequence of the invention, e. g., SEQ ID NO : 1, SEQ ID NO : 2, or SEQ ID NO : 3, over a region of at least about 100 residues, 150 residues, 200 residues or more, or a region ranging from between about 50 residues or 100 residues to the full length of the nucleic acid or polypeptide. Nucleic acid sequences of the invention can be substantially identical over the entire length of a polypeptide coding region.

Additionally a "substantially identical" amino acid sequence is a sequence that differs from a reference sequence by one or more conservative or non-conservative amino acid substitutions, deletions, or insertions, particularly when such a substitution occurs at a site that is not the active site of the molecule, and provided that the polypeptide essentially retains its functional properties. A conservative amino acid substitution, for example, substitutes one amino acid for another of the same class (e. g., substitution of one hydrophobic amino acid, such as isoleucine, valine, leucine, or methionine, for another, or substitution of one polar amino acid for another, such as substitution of arginine for lysine). One or more amino acids can be deleted, for example, from a phospholipase polypeptide, resulting in modification of the structure of the polypeptide, without significantly altering its biological activity. For example, amino-or carboxyl-terminal amino acids that are not required for phospholipase (biological) activity can be removed. Modified polypeptide sequences of the invention can be assayed for phospholipase biological activity by any number of methods, including contacting the modified polypeptide sequence with a phospholipase substrate and determining whether the modified polypeptide decreases the amount of specific substrate in the assay or increases the bioproducts of the enzymatic reaction of a functional phospholipase with the substrate.

"Hybridization" refers to the process by which a nucleic acid strand joins with a complementary strand through base pairing. Hybridization reactions can be sensitive and selective so that a particular sequence of interest can be identified even in samples in which it is present at low concentrations. Suitably stringent conditions can be defined by, for example, the concentrations of salt or formamide in the prehybridization and hybridization solutions, or by the hybridization temperature, and are well known in the art. For example, stringency can be increased by reducing the concentration of salt, increasing the concentration of formamide, or raising the hybridization temperature, altering the time of hybridization, as described in detail, below. In alternative aspects, nucleic acids of the invention are defined by their ability to hybridize under various stringency conditions (e. g., high, medium, and low), as set forth in WO03/089620 A2 on pages 53 and 59, explicitly incorporated herein by reference. Nucleic acids shorter than full length are also included. These nucleic acids are also useful as, e. g., hybridization probes, labelling probes, PCR oligonucleotide probes, iRNA (single or double stranded), antisense or sequences encoding antibody binding peptides (epitopes), motifs, active sites and the like. In one aspect, nucleic acids of the invention are defined by their ability to hybridize under high stringency conditions comprising conditions at 42 °C in 50% formamide , 5X SSPE, 0.3% SDS, and a repetitive sequence blocking nucleic acid, such as salmon sperm DNA (see e.g. Sambrook, ed., MOLECULAR CLONING: A LABORATORY MANUAL (2ND ED.), Vols. 1-3, Cold Spring Harbor Laboratory,(1989) ; CURRENT PROTOCOLS IN MOLECULAR BIOLOGY, Ausubel, ed. John Wiley & Sons, Inc., New York (1997) ; LABORATORY TECHNIQUES IN BIOCHEMISTRY AND MOLECULAR BIOLOGY: HYBRIDIZATION WITH NUCLEIC ACID PROBES, Partl. Theory and Nucleic Acid Preparation,Tijssen, ed. Elsevier, N. Y. (1993).). The temperature range corresponding to a particular level of stringency can be further narrowed by calculating the purine to pyrimidine ratio of the nucleic acid of interest and adjusting the temperature accordingly. Nucleic acids of the invention are also defined by their ability to hybridize under high, medium, and low stringency conditions as set forth in Ausubel and Sambrook (see above). Variations on the above ranges and conditions are well known in the art.

The term "variant" refers to polynucleotides or polypeptides of the invention modified at one or more base pairs, codons, introns, exons, or amino acid residues (respectively) yet still retain the biological activity of a phospholipase of the invention.

Variants can be produced by any number of means including methods such as, for example, error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, GSSM and any combination thereof. Such techniques may in particular be employed to introduce a codon encoding the aspartate residue discussed above into a nucleic acid sequence encoding a phospholipase not comprising such aspartate residue in order to increase the thermostability of the phospholipase.

The term "saturation mutagenesis" or "GSSM" includes a method that uses degenerate oligonucleotide primers to introduce point mutations into a polynucleotide; the term "optimized directed evolution system "or "optimized directed evolution" includes a method for reassembling fragments of related nucleic acid sequences, e. g. , related genes; the term "synthetic ligation reassembly" or "SLR" includes a method of ligating oligonucleotide fragments in a non-stochastic fashion (as described e.g. in WO 03/089620 A2)..

### Generating and Manipulating Nucleic Acids

The nucleic acids of the invention can be made, isolated and/or manipulated by, e. g., cloning and expression of cDNA libraries, amplification of message or genomic DNA by PCR, and the like. In practicing the methods of the invention, homologous genes can be modified by manipulating a template nucleic acid, as described herein. The invention can be practiced in conjunction with any method or protocol or device known in the art, which are well described in the scientific and patent literature.

Suitable general techniques are for example described in WO 03/089620 A2. Its respective disclosure is herewith explicitly incorporated by reference. In summary, the nucleic acids used to practice this invention, whether RNA, iRNA, antisense nucleic acid, cDNA, genomic DNA, vectors, viruses or hybrids thereof, may be isolated from a variety of sources, genetically engineered, amplified, and/or expressed/ generated recombinantly. Recombinant polypeptides generated from these nucleic acids can be individually isolated or cloned and tested for a desired activity. Any recombinant expression system can be used, including bacterial, mammalian, yeast, insect or plant cell expression systems.

Alternatively, these nucleic acids can be synthesized in vitro by well-known chemical synthesis techniques, as described in, e. g. , Adams (1983) J. Am. Chem. Soc. 105: 661; Belousov (1997) Nucleic Acids Res. 25: 3440-3444 ; Frenkel (1995) Free Radic. Biol. Med. 19: 373-380; Blommers (1994) Biochemistry 33: 7886-7896; Narang (1979) Meth. Enzymol. 68: 90; Brown (1979) Meth. Enzymol. 68: 109; Beaucage (1981) Tetra. Lett. 22: 1859; U. S. Patent No. 4,458, 066.

Techniques for the manipulation of nucleic acids, such as, e.g., subcloning, labelling probes (e.g., random-primer labelling using Klenow polymerase, nick translation, amplification), sequencing, hybridization and the like are well described in the scientific and patent literature, see, e. g. , Sambrook, ed., MOLECULAR CLONING: A LABORATORY MANUAL (2ND ED.), Vols. 1-3, Cold Spring Harbor Laboratory,(1989) ; CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Partl. Theory and Nucleic Acid Preparation,Tijssen, ed. Elsevier, N. Y. (1993).

Another useful means of obtaining and manipulating nucleic acids used to practice the methods of the invention is to clone from genomic samples, and, if desired, screen and re-clone inserts isolated or amplified from, e. g., genomic clones or cDNA clones. Sources of nucleic acid used in the methods of the invention include genomic or cDNA libraries contained in, e. g., mammalian artificial chromosomes (MACs), see, e. g. , U. S. Patent Nos. 5,721, 118; 6,025, 155; human artificial chromosomes, see, e. g. , Rosenfeld (1997) Nat. Genet. 15: 333-335; yeast artificial chromosomes (YAC); bacterial artificial chromosomes (BAC); PI artificial chromosomes, see, e. g. , Woon (1998) Genomics 50: 306-316; Pl-derived vectors (PACs), see, e. g., Kern (1997) Biotechniques 23: 120-124; cosmids, recombinant viruses, phages or plasmids. In one aspect, a nucleic acid encoding a polypeptide of the invention is assembled in appropriate phase with a leader sequence capable of directing secretion of the translated polypeptide or fragment thereof.

According to one embodiment, the present invention provides fusion proteins and nucleic acids encoding them. A polypeptide of the invention can be fused to a heterologous peptide or polypeptide, such as N-terminal identification peptides which impart desired characteristics, such as increased stability or simplified purification. Peptides and polypeptides of the invention can also be synthesized and expressed as fusion proteins with one or more additional domains linked thereto for, e. g., producing a more immunogenic peptide, to more readily isolate a recombinantly synthesized peptide, to identify and isolate antibodies and antibody-expressing B cells, and the like. Detection and purification facilitating domains include, e. g., metal chelating peptides such as polyhistidine tracts and histidine-tryptophan modules that allow purification on immobilized metals, protein A domains that allow purification on immobilized immunoglobulin, and the domain utilized in the FLAGSextension/affinity purification system (Immunex Corp, US). The inclusion of a cleavable linker sequence such as Factor Xa or enterokinase (Invitrogen, US) between a purification domain and the motif-comprising peptide or polypeptide facilitates purification.

For example, an expression vector can include an epitope-encoding nucleic acid sequence linked to six histidine residues followed by a thioredoxin and an enterokinase cleavage site (see e. g. , Williams (1995) Biochemistry 34: 1787-1797; Dobeli (1998) Protein Expr. Purif. 12: 404-414). The histidine residues facilitate detection and purification while the enterokinase cleavage site provides a means for purifying the epitope from the remainder of the fusion protein. Technology pertaining to vectors encoding fusion proteins and application of fusion proteins are well described in the scientific and patent literature, see e. g., Kroll (1993) DNA Cell. Biol.,12 : 441-53.

Transcriptional and translational control sequences: The invention provides nucleic acid (e. g., DNA) sequences operatively linked to expression (e. g., transcriptional or translational) control sequence (s),. e. g., promoters or enhancers, to direct or modulate RNA synthesis/expression. The expression control sequence can be in an expression vector. Exemplary bacterial promoters include lacl, lacZ, T3, T7, gpt, lambda PR, PL and trp. Exemplary eukaryotic promoters include CMV immediate early, HSV thymidine kinase, early and late SV40, LTRs from retrovirus, and mouse metallothioneinl.

Promoters suitable for expressing a polypeptide in bacteria include the E. coli lac or trp promoters, the lacl promoter, the lacZ promoter, the T3 promoter, the T7 promoter, the gpt promoter, the lambda PR promoter, the lambda PL promoter, promoters from operons encoding glycolytic enzymes such as3-phosphoglycerate kinase (PGK), and the acid phosphatase promoter. Eukaryotic promoters include the CMV immediate early promoter, the HSV thymidine kinase promoter, heat shock promoters, the early and late SV40 promoter, LTRs from retroviruses, and the mouse metallothionein-I promoter. Other promoters known to control expression of genes in prokaryotic or eukaryotic cells or their viruses may also be used. A prefered promoter is the inducible acoA promoter sequence from B. subtilis as present in pMSE3 (see below). A preferred signal peptide sequence is the amyE sequence from B. subtilis as present in pMSE3 (see below). The acoA and amyE sequences are known e.g. from Kunst, F. et al., Nature 390 (6657), 249-256 (1997).

Expression vectors and cloning vehicles: The invention provides expression vectors and cloning vehicles comprising nucleic acids of the invention, e. g., sequences encoding the phospholipases of the invention. Expression vectors and cloning vehicles of the invention can comprise viral particles, baculovirus, phage, plasmids, phagemids, cosmids, fosmids, bacterial artificial chromosomes, viral DNA (e. g., vaccina, adenovirus, fowl pox virus, pseudorabies and derivatives of SV40), PI-based artificial chromosomes, yeast plasmids, yeast artificial chromosomes, and any other vectors specific for specific hosts of interest (such as bacillus, Aspergillus and yeast).

Vectors of the invention can include chromosomal, non-chromosomal and synthetic DNA sequences. Large numbers of suitable vectors are known to those of skill in the art, and are commercially available. Exemplary vectors are include: bacterial: pQE vectors (Qiagen), pBluescript plasmids, pNH vectors, (lambda-ZAP vectors (Stratagene); ptrc99a,pKK223-3, pDR540, pRIT2T (Pharmacia) ; Eukaryotic:pXTI, pSG5 (Stratagene), pSVK3, pBPV,pMSG, pSVLSV40(Pharmacia). However, any other plasmid or other vector may be used so long as they are replicable and viable in the host. Low copy number or high copy number vectors may be employed with the present invention.

The expression vector may comprise a promoter, a ribosome-binding site for translation initiation and a transcription terminator. The vector may also include appropriate sequences for amplifying expression. Mammalian expression vectors can comprise an origin of replication, any necessary ribosome binding sites, a polyadenylation site, splice donor and acceptor sites, transcriptional termination sequences, and 5'flanking non-transcribed sequences. In some aspects, DNA sequences derived from the SV40 splice and polyadenylation sites may be used to provide the required non-transcribed genetic elements.

In one aspect, the expression vectors contain one or more selectable marker genes to permit selection of host cells containing the vector. Such selectable markers include genes encoding dihydrofolate reductase or genes conferring neomycin resistance for eukaryotic cell culture, genes conferring tetracycline or ampicillin resistance in E. coli, and the S. cerevisiae TRP1 gene. Promoter regions can be selected from any desired gene using chloramphenicol transferase (CAT) vectors or other vectors with selectable markers.

Vectors for expressing the polypeptide or fragment thereof in eukaryotic cells may also contain enhancers to increase expression levels. Enhancers are cis-acting elements of DNA, usually from about 10 to about 300 bp in length that act on a promoter to increase its transcription. Examples include the SV40 enhancer on the late side of the replication origin bp 100 to 270, the cytomegalovirus early promoter enhancer, the polyoma enhancer on the late side of the replication origin, and the adenovirus enhancers.

A DNA sequence may be inserted into a vector by a variety of procedures. In general, the DNA sequence is ligated to the desired position in the vector following digestion of the insert and the vector with appropriate restriction endonucleases. Alternatively, blunt ends in both the insert and the vector may be ligated. A variety of cloning techniques are known in the art, e. g. , as described in Ausubel and Sambrook. Such procedures and others are deemed to be within the scope of those skilled in the art.

The vector may be in the form of a plasmid, a viral particle, or a phage. Other vectors include chromosomal, non-chromosomal and synthetic DNA sequences, derivatives of SV40; bacterial plasmids, phage DNA, baculovirus, yeast plasmids, vectors derived from combinations of plasmids and phage DNA, viral DNA such as vaccina, adenovirus, fowl pox virus, and pseudorabies. A variety of cloning and expression vectors for use with prokaryotic and eukaryotic hosts are described by, e. g., Sambrook (see above).

Particular bacterial vectors which may be used include the commercially available plasmids comprising genetic elements of the well known cloning vector pBR322 (ATCC 37017), pKK223-3 (Pharmacia Fine. Chemicals, Uppsala, Sweden), GEM1 (Promega Biotec, Madison, WI, USA) pQE70, pQE60, pQE-9 (Qiagen), pD10, psiX174pBluescriptll KS, pNH8A, pNH16a, pNH18A, pNH46A (Stratagene), ptrc99a, pKK223-3, pKK233-3, pDR540, pRIT5(Phanmacia), pKK232-8 and pCM7. Particular eukaryotic vectors include pSV2CAT, pOG44,pXTI, pSG (Stratagene) pSVK3,pBPV,pMSG, and pSVL(Pharmacia). However, any other vector may be used as long as it is replicable and viable in the host cell. A suitable PLC encoding insert for cloning in those vectors is e.g. UniProtKB/TrEMBL data base entry Q63FW8. A preferred cloning vector is the TOPO® vector (Invitrogen, Carlsbad, USA). A preferred expression vector is the pMSE vector (see Examples below).

Host cells and transformed cells: The invention also provides a transformed cell comprising a nucleic acid sequence of the invention, e. g., a sequence encoding a phospholipase of the invention, a vector of the invention. The host cell may be any of the host cells familiar to those skilled in the art, including prokaryotic cells, eukaryotic cells, such as bacterial cells, fungal cells, yeast cells, mammalian cells, insect cells, or plant cells. Exemplary bacterial cells include E. coli, Streptomyces, Bacillus subtilis, Salmonella typhimurium and various species within the genera Pseudomonas, Streptomyces, and Staphylococcus. Exemplary insect cells include Drosophila S2 and Spodoptera Sf9. Exemplary animal cells include CHO, COS or Bowes melanoma or any mouse or human cell line. The selection of an appropriate host is within the abilities of those skilled in the art.

The vector may be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation (Davis, L. , Dibner, M., Battey,l., Basic Methods in Molecular Biology, (1986)).

Where appropriate, the engineered host cells can be cultured in conventional nutrient media modified as appropriate for activating promoters, selecting transformants or amplifying the genes of the invention. Following transformation of a suitable host strain and growth of the host strain to an appropriate cell density, the selected promoter may be induced by appropriate means (e. g., temperature shift or chemical induction) and the cells may be cultured for an additional period to allow them to produce the desired polypeptide or fragment thereof.

Cells can be harvested by centrifugation, disrupted by physical or chemical means, and the resulting crude extract is retained for further purification. Microbial cells employed for expression of proteins can be disrupted by any convenient method, including freeze-thaw cycling, sonification, mechanical disruption, or use of cell lysing agents. Such methods are well known to those skilled in the art. The expressed polypeptide or fragment thereof can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide. If desired, high performance liquid chromatography (HPLC) can be employed for final purification steps.

Various mammalian cell culture systems can also be employed to express recombinant protein. Examples of mammalian expression systems include the COS-7 lines of monkey kidney fibroblasts and other cell lines capable of expressing proteins from a compatible vector, such as the C127, 3T3, CHO, HeLa and BHK cell lines.

The constructs in host cells can be used in a conventional manner to produce the gene product encoded by the recombinant sequence. Depending upon the host employed in a recombinant production procedure, the polypeptides produced by host cells containing the vector may be glycosylated or may be non-glycosylated. Polypeptides of the invention may or may not also include an initial methionine amino acid residue.

Cell-free translation systems can also be employed to produce a polypeptide of the invention. Cell-free translation systems can use mRNAs transcribed from a DNA construct comprising a promoter operably linked to a nucleic acid encoding the polypeptide or fragment thereof. In some aspects, the DNA construct may be linearized prior to conducting an in vitro transcription reaction. The transcribed mRNA is then incubated with an appropriate cell-free translation extract, such as a rabbit reticulocyte extract, to produce the desired polypeptide or fragment thereof.

The expression vectors can contain one or more selectable marker genes to provide a phenotypic trait for selection of transformed host cells such as dihydrofolate reductase or neomycin resistance for eukaryotic cell culture, or such as tetracycline or ampicillin resistance in E. coli. A preferred host cell is *B. subtilis.*

Amplification of Nucleic Acids: In practicing the invention, nucleic acids encoding the polypeptides of the invention, or modified nucleic acids, can be reproduced by, e. g., amplification. The invention provides amplification primer sequence pairs for amplifying nucleic acids encoding polypeptides with a phospholipase activity. In one aspect, the primer pairs are capable of amplifying nucleic acid sequences of the invention, e. g. , including the exemplary SEQ ID NO : 3, or a subsequence thereof. One of skill in the art can design amplification primer sequence pairs for any part of or the full length of these sequences.

The invention provides an amplification primer sequence pair for amplifying a nucleic acid encoding a polypeptide having a phospholipase activity, wherein the primer pair is capable of amplifying a nucleic acid comprising a sequence of the invention, or fragments or subsequences thereof. One or each member of the amplification primer sequence pair can comprise an oligonucleotide comprising at least about 10 to 50 consecutive bases of the sequence, or about 12, 13, 14, 15, 16, 17, 18, 19, 20, 21, 22, 23, 24, or 25 consecutive bases of the sequence. The invention provides amplification primer pairs, wherein the primer pair comprises a first member having a sequence as set forth by about the first (the 5') 12, 13, 14, 15, 16, 17, 18, 19, 20, 21, 22, 23, 24, or 25 residues of a nucleic acid of the invention, and a second member having a sequence as set forth by about the first (the 5') 12, 13, 14, 15, 16, 17, 18, 19, 20, 21, 22, 23, 24, or 25 residues of the complementary strand of the first member.

The invention provides phospholipases (i.e. nucleic acid sequences encoding phospholipases) generated by amplification, e. g., polymerase chain reaction (PCR), using an amplification primer pair of the invention. The invention provides methods of making a phospholipase by amplification, e. g. , polymerase chain reaction (PCR), using an amplification primer pair of the invention. In one aspect, the amplification primer pair amplifies a nucleic acid from a library, e. g., a gene library, such as an environmental library. Amplification reactions can also be used to quantify the amount of nucleic acid in a sample (such as the amount of message in a cell sample), label the nucleic acid (e. g., to apply it to an array or a blot), detect the nucleic acid, or quantify the amount of a specific nucleic acid in a sample. In one aspect of the invention, messages isolated from a cell or acDNA library are amplified. The skilled artisan can select and design suitable oligonucleotide amplification primers. Amplification methods are also well known in the art, and include, e.g., polymerase chain reaction, PCR (see, e. g. , PCR PROTOCOLS, A GUIDE TO METHODS AND APPLICATIONS, ed. unis, Academic Press, N. Y. (1990) and PCR STRATEGIES (1995), ed.hinis, Academic Press, Inc., N. Y. , ligase chain reaction (LCR) (see, e. g. , Wu (1989) Genomics 4: 560; Landegren (1988) Science 241:1077 ; Barringer (1990) Gene 89: 117); transcription amplification (see, e. g. , Kwoh (1989) Proc. Natl. Acad. Sci. USA 86: 1173); and, self-sustained sequence replication (see, e. g. , Guatelli (1990) Proc. Natl. Acad. Sci. USA 87: 1874); Q Beta replicase amplification (see, e.g., Smith (1997) J. Clin. Microbiol.35 :1477-1491), automated Q-beta replicase amplification assay (see, e. g., Burg (1996) Mol. Cell. Probes 10: 257-271) and other RNA polymerase mediated techniques (e. g., NASBA, Cangene, Mississauga, Ontario); see also Berger (1987) Methods Enzymol. 152: 307-316; Sambrook; Ausubel; U. S. Patent Nos. 4,683, 195 and 4,683, 202; Sooknanan (1995) Biotechnology 13: 563-564.

Determining the degree of sequence identity: The extent of sequence identity (homology) may be determined using any computer program and associated parameters, including those described herein, such as BLAST 2.2. 2. or FASTA version 3. 0t78, with the default parameters.

In alternative embodiments, the sequence identity can be over a region of at least about 5, 10, 20, 30, 40, 50, 100, 150, 200 or more consecutive residues, or the full length of the nucleic acid or polypeptide. The extent of sequence identity (homology) may be determined using any computer program and associated parameters, including those described herein, such as-BLAST 2.2. 2. or FASTA version 3.0t78, with the default parameters. Such algorithms and programs include, but are not limited to, TBLASTN, BLASTP, FASTA, TFASTA, and CLUSTALW (Pearson and Lipman, Proc. Natl. Acad. Sci. USA 85 (8): 2444-2448,1988 ; Altschul et al. , J. Mol. Biol. 215 (3): 403-410, 1990 ; Thompson etal., Nucleic Acids Res. 22 (2): 4673-4680, 1994 ; Higgins et al., Methods Enzymol. 266: 383- 402,1996 ; Altschul et al. , J. Mol. Biol. 215 (3): 403-410,1990 ; Altschul et al., Nature Genetics 3: 266-272,1993). Homologous sequences also include RNA sequences in which uridines replace the thymines in the nucleic acid sequences. The homologous sequences may be obtained using any of the procedures described herein or may result from the correction of a sequencing error. It will be appreciated that the nucleic acid sequences as set forth herein can be represented in the traditional single character format (see, e. g., Stryer, Lubert. Biochemistry, 3rd Ed.,W. H Freeman & Co. , New York) or in any other format which records the identity of the nucleotides in a sequence. Alternatively to the use of computer programs, the sequence comparison may also be done by optical inspection/comparison or by one of the methods disclosed in WO03/089620 A2 on pages 41 to 53.

As used herein, the term "aspartate" is also used to encompass "aspartic acid".

As used herein, the term "phospholipase" is also used to encompass "polypeptide having phospholipase activity". The polypeptide preferably is an isolated or recombinant polypeptide.

According to a preferred embodiment, the phospholipase used according to the invention is a polypeptide comprising SEQ ID NO : 2 or a sequence having at least 75% sequence identity to SEQ ID NO : 2 over a region of at least about 100 residues, wherein the sequence identities are determined by analysis with a sequence comparison algorithm or by a visual inspection, the polypeptide comprising the aspartate residue corresponding to position 174 of SEQ ID NO: 2. The preferred mode of sequence comparison has been described above. According to a further preferred embodiment, the phospholipase used has a sequence identical or substantially identical to a region of 5, 7, 10, 15, 20, 25, 30, 35, 40, 45 or 50 amino acids both upstream and downstream (i.e. on both sides) the aspartate in position 174 of SEQ ID NO:2.

According to one aspect of the invention, a preferred polypeptide having phospholipase activity and comprising the aspartate residue as set out above is the polypeptide comprising SEQ ID NO : 2. Also, based on SEQ ID NO : 1 and SEQ ID NO : 2, respectively, and the above surprising finding of the role of the aspartate residue, the skilled person may easily identify other polypeptides with phospholipase activity using an oligonucleotide encoding all or part of SEQ ID NO:1 as a screening or hybridisation probe. Also, oligonucleotides encoding parts of or the complete SEQ ID NO : 2 may be used for this purpose. Those oligonucleotide probes may be used for screening genomic or cDNA libraries by standard methods known to the skilled person. According to a preferred embodiment, the oligonucleotide used for screening or hybridisation comprises the codon encoding the aspartate residue as set out above.

According to a further aspect of the invention use is made of an isolated or recombinant polypeptide encoded by a nucleic acid comprising SEQ ID NO : 3 or a sequence having at least 75% sequence identity to SEQ ID NO : 3 over a region of at least about 100 residues, wherein the sequence identities are determined by analysis with a sequence comparison algorithm or by a visual inspection, or encoded by a sequence hybridizing to SEQ ID NO : 3 under stringent or highly stringent conditions. The modes of preferred sequence comparison and hybridisation have been described above. According to a preferred embodiment, the stringent conditions include a wash step comprising a wash in 0. 2X SSC at a temperature of about 65 °C for about 15 minutes.

Regarding the sequences showing at least 75% or sequence identity to SEQ ID NO : 1, SEQ ID NO : 2 or SEQ ID NO : 3, respectively, it is further preferred that the sequence identity is at least about 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or is 100% sequence identity.

Also, it is further preferred that the sequence identity is over a region of at least about 10, 15, 20, 25, 30, 35, 40, 45, 50, 75, 100, 150, 200, 250, 300, 350, 400, 450, 500, 550, 600, 650, 700, 800, 900, 1000 or the full length of SEQ ID 3 or over a region of at least about 10, 15, 20, 25, 30, 35, 40, 45, 50, 75, 100, 150, 200, 250, or the full length of SEQ ID NO 2, respectively.

According to the invention, the polypeptide used has a phospholipase activity as set out above. Preferably, the phospholipase activity comprises catalyzing hydrolysis of a phosphoric acid diester linkage. It is further preferred that the phospholipase activity comprises a phospholipase C (PLC) activity (EC 3.1.4.3) Thus, unlike PLAs or PLBs, the activity of PLCs on phospholipids does not lead to free fatty acids which may be of disadvantage in the preparation of degummed oil of fat, but results in phosphomonoesters which can easily be removed together with the aqueous phase in the degumming of oil or fat.

According to a further preferred aspect of the invention, the phospholipase activity comprises a PC-PLC activity, i.e. a phosphatidylcholine-specific phospholipase C activity [E.C.3.1.4.3]. It is preferred that the polypeptide used according to the invention is capable of catalyzing hydrolysis of an ester linkage in a phospholipid in a vegetable oil. The vegetable oil phospholipid comprises preferably an oilseed phospholipid. Furthermore, the vegetable oil phospholipid is preferably, but not obligatorily, derived from a plant oil, a high phosphorous oil, a soy oil, a canola oil, a palm oil, a cottonseed oil, a corn oil, a palm kernel-derived phospholipid, a coconut oil, a peanut oil, a sesame oil, a fish oil, an algae phospholipid, a sunflower oil, an essential oil, a fruit seed oil, a grapeseed phospholipid, an apricot phospholipid, or a borage phospholipid.

As explained above, it has been surprisingly found that the polypeptides of the invention also show a phospholipase activity at elevated temperatures. The phospholipase activity of the polypeptide is therefore preferably thermotolerant or thermostable. According to a preferred embodiment, this means that the isolated or recombinant polypeptide retains a phospholipase activity under conditions comprising a temperature above 37 °C, preferably above 50 °C, more preferably above 60 °C, more preferably above 70 °C, more preferably above 80 °C. Further, the polypeptide of the invention preferably shows a phospholipase activity in the range of between about 37 to about 95 °C, preferably between about 55 to about 85 °C, between about 60 to about 95°C, between about 65 °C to about 75 °C, or between about 90 C to about 95 °C.

According to a more preferred embodiment, the phospholipase used has a maximum of its phospholipase activity at a temperature above 50 °C, preferably above 55°C, more preferably above 60 °C. The conditions for testing the phospholipase activity are as set out above, particularly as set out in the Examples below (NPPC assay for PLC activity). Thus, the phospholipase activity of the polypeptide of the invention preferably comprises a specific activity at 60 °C (in the NPPC assay described below) of at least about 10 preferably at least about 50 or at least 100, preferably at least about 200, more preferably at least about 500 units per milligram of protein, more preferably at least about 1000 units per milligram of protein, more preferably at least about 5000 units per milligram of protein, more preferably at least about 10000 units per milligram of protein.

Further preferred, the polypeptide has a phospholipase activity at a temperature of 70 °C which is at least 50% of its maximum phospholipase activity, i.e. the phospholipase activity at the temperature optimum. Thus, the phospholipase activity of the polypeptide in question is determined at different temperatures using the NPPC assay as described in the Examples below. The temperature at which the highest phospholipase activity (maximum phospholipase activity) is measured is considered as the temperature optimum. The phospholipase activity at a temperature of 70 °C is then compared to the above maximum phospholipase activity and should be 50% thereof or more according to a preferred embodiment of the invention.

The isolated or recombinant polypeptide preferably has a maximum of its phospholipase activity at a pH in the range of 4 to 6, preferably in the range of 4,5 to 6. However, the polypeptide may also be used at a pH outside this preferred range.

Many phospholipases, like for example the PC-PLC of B. cereus are extracellular proteins. They are e.g. synthesized as a pre-proenzyme from which a presequence and a prosequence are cleaved to obtain the mature polypeptide/enzyme. According to one embodiment of the invention, the (isolated or recombinant) polypeptide used lacks a pre- or prosequence, and the nucleic acid encoding the polypeptide lacks a signal sequence. The absence or presence of signal sequences and pre- or prosequences will depend on the particular expression system chosen, as is evident to the skilled person. Thus, if intracellular transport and secretion of the polypeptide of the invention is desired, any of the known signal sequences, pre- or prosequences operable in the host cell chosen may be used. Commercial expression vectors for overexpression by certain host cells commonly contain already suitable sequences in their expression cassettes. For example, heterologous signal sequence most effective in the expression system/host cells used may be employed. E.g. the Csn-, GlpQ and AmyE signal peptide sequences have been found to be useful.

As described above, according to one embodiment of the present invention, the isolated or recombinant polypeptide used is immobilized on a solid carrier. Suitable carriers include for example a cell, a metal, a resin, a polymer, a ceramic, a glass, a microelectrode, a graphitic particle, a bead, a gel, a plate, an array or a capillary tube, depending on the intended application of the polypeptide of the invention. The polypeptides, fragments thereof or nucleic acids encoding them can be affixed to a solid support by methods known in the art as e.g. listed in WO 03/089620 A2 on pages 111 to 115, explicitly incorporated herein by reference. Thus, immobilisation is often economical and efficient in the use of the phospholipases in industrial processes. For example, a consortium or cocktail of phospholipase enzymes (or active fragments thereof), which are used in a specific chemical reaction, can be attached to a solid support and dunked into a process vat. The enzymatic reaction can occur. Then, the solid support can be taken out of the vat, along with the enzymes affixed thereto, for repeated use. In one embodiment of the invention, an isolated nucleic acid of the invention is affixed to a solid support. In another embodiment of the invention, the solid support is selected from the group of a gel, a resin, a polymer, a ceramic, a glass, a microelectrode and any combination thereof. For example, solid supports useful in this invention include gels. Some examples of gels include Sepharose, gelatin, glutaraldehyde, chitosan-treated glutaraldehyde, albumin-glutaraldehyde, chitosan-Xanthan, toyopearl gel (polymer gel), alginate, alginate- polylysine, carrageenan, agarose, glyoxyl agarose, magnetic agarose, dextran-agarose, poly (Carbamoyl Sulfonate) hydrogel, BSA-PEG hydrogel, phosphorylated polyvinyl alcohol (PVA), monoaminoethyl-N-aminoethyl (MANA), amino, or any combination thereof. Another solid support useful in the present invention are resins or polymers. Some examples of resins or polymers include cellulose, acrylamide, nylon, rayon, polyester, anion-exchange resin, AMBERLITE^{™} XAD-7, AMBERLITE^{™}XAD-8, AMBERLITE^{™} IRA-94, AMBERLITE^{™} IRC-50, polyvinyl, polyacrylic, polymethacrylate, or any combination thereof. Another type of solid support useful in the present invention is ceramic. Some examples include non-porous ceramic, porous ceramic, SiO₂, Al₂O₃. Another type of solid support useful in the present invention is glass. Some examples include non-porous glass, porous glass, aminopropyl glass or any combination thereof. Another type of solid support that can be used is a microelectrode. An example is a polyethyleneimine-coated magnetite. Graphitic particles can be used as a solid support. Another example of a solid support is a cell, such as a red blood cell.

There are many methods that would be known to one of skill in the art for immobilizing enzymes or fragments thereof, or nucleic acids, onto a solid support. Some examples of such methods include, e. g., electrostatic droplet generation, electrochemical means, via adsorption, via covalent binding, via cross-linking, via a chemical reaction or process, via encapsulation, via entrapment, via calcium alginate, or via poly (2-hydroxyethyl methacrylate). Like methods are described in Methods in Enzymology, Immobilized Enzymes and Cells, Part C.1987. Academic Press. Edited by S. P. Colowick and N.O. Kaplan. Volume 136 ; and Immobilization of Enzymes and Cells. 1997. Humana Press. Edited by G. F.Bickerstaff. Series: Methods in Biotechnology, Edited by J. M. Walker.

According to a preferred embodiment of the invention, the polypeptide is immobilized by contacting it with a clay or a layer silicate, preferably a phyllosilicate as a bentonite, a bentonite derivative or an attapulgite or hectorite, in particular an activated layer silicate, commonly referred to as bleaching earth. Other preferred carrier materials are natural or synthetic smectites; acid-activated smectites, particularly bentonites; alkali-activated smectites, particularly bentonites; organically modified smectites, particularly bentonites; or hydrotalcites. This has the advantage of combining two functional components used in the treatment/refining of crude oil of fat.

As already mentioned above, according to a further aspect of the invention an isolated or recombinant nucleic acid encoding for a polypeptide as described herein is used to express the polypeptide or to screen nucleic acid libraries for other suitable polypeptides containing SEQ ID No:1. According to a preferred embodiment, the isolated or recombinant nucleic acid comprises SEQ ID NO : 3 or a sequence having at least 75% sequence identity to SEQ ID NO : 3 over a region of at least about 100 residues, wherein the nucleic acid encodes at least one polypeptide having a phospholipase activity, and the sequence identities are determined by analysis with a sequence comparison algorithm or by a visual inspection. The sequence identity is preferably at least about 76%, 77%, 78%, 79%, 80%, 81 %, 82%, 83%, 84%, 85%, 86%, 87%, 88%, 89%, 90%, 91 %, 92%, 93%, 94%, 95%, 96%,97%, 98%, 99%, or more, or is 100% sequence identity to SEQ ID NO : 3; and the the sequence identity is preferably over a region of at least about 50, 75, 100,150, 200, 250, 300, 350, 400, 450, 500, 550, 600, 650, 700, 750, 800, 850, 900, 950, 1000, or the full length of SEQ ID NO : 3.

According to a further aspect of the invention, the isolated or recombinant nucleic acid is integrated in a construct, in particular a bacterial or viral vector, a plasmid, a phage, a phagemid, a cosmid, a fosmid, a bacteriophage or an artificial chromosome. Preferably, the construct is an expression vector.

In such a vector or expression vector the nucleic acid molecule preferably is operably linked to an expression cassette, including a promoter allowing the expression of the polypeptide encoded by the nucleic acid molecule.

According to a more preferred embodiment of the present invention it has been found that a high overexpression of the polypeptides of the invention can be achieved using an inducible promoter, in particular an acoAamyE promoter sequence. The expression of the polypeptide in such a vector can be induced by adding acetoin to the culture medium of the host cells transformed with the vector at a final concentration of e.g. 0.5 %.

Thus, according to a further aspect of the present invention a transformed cell comprising a nucleic acid molecule or a construct as described herein is used. According to a preferred embodiment, bacterial cells are used as host cells i.e. for transformation with the nucleic acid or vector containing the same. Most preferable, *B. subtilis* cells are used for expression, particularly of bacterial PLCs, in particular those originating from *B. cereus.* The advantage of this system is that *B. subtilis* and *B. cereus* are closely related and B. subtilis is non-pathogenic. Therefore the expression is nearly homologue. Also, the expression system was found to avoid the common occurrence of inclusion bodies containing the polypeptide of the invention, which have to be purified, solubilized and refolded with great effort.

A further aspect of the present invention is directed to a method for identifying a polypeptide having a phospholipase activity comprising the following steps: (a) providing a polypeptide as set forth herein; (b) providing a phospholipase substrate; and (c) contacting the polypeptide with the substrate of step (b) and detecting a decrease in the amount of substrate or an increase in the amount of a reaction product, wherein a decrease in the amount of the substrate or an increase in the amount of the reaction product detects a polypeptide having a phospholipase activity. As a phospholipase substrate any natural or synthetic substrate may be used, including e.g. phosphatidylcholine, phosphatidylinositol-4,5-bisphosphate or NPPC.

Yet another aspect of the present invention is directed to a method for isolating or recovering a nucleic acid encoding a polypeptide with a phospholipase activity from an environmental sample comprising the steps of: (a) providing an amplification primer sequence pair, wherein each member of the amplification primer sequence pair comprises an oligonucleotide comprising at least about 10 to 50 consecutive bases of a sequence as set forth in SEQ ID NO : 3, (b) isolating a nucleic acid from the environmental sample or treating the environmental sample such that nucleic acid in the sample is accessible for hybridization to the amplification primer pair; and, (c) combining the nucleic acid of step (b) with the amplification primer pair of step (a) and amplifying nucleic acid from the environmental sample, thereby isolating or recovering a nucleic acid encoding a polypeptide with a phospholipase activity from an environmental sample. A non-limiting example of a suitable pair of amplification primers is: FW-mPLC: 5'-TGG TCT GCT GAA GAT AAA CAT AAA GAA GG-3', PLC-RW-Xba: 5'-CCG TCT AGA TTA ACG ATC TCC GTA CG-3'.

Another aspect of the present invention is directed to a method of increasing thermotolerance or thermostability of a phospholipase polypeptide, the method comprising the steps of:
(a) providing the nucleic acid sequence encoding the phospholipase polypeptide;
(b) identifying the part of the nucleic acid sequence encoding SEQ ID 4: TNPEXWIHG, wherein X is any amino acid other than aspartate;
(c) modifying one or more nucleotides in the codon encoding amino acid X so that the codon for the amino acid aspartate results.

Thus, the surprising finding of the inventors regarding the importance of the aspartate amino acid residue in SEQ ID NO : 1 and SEQ ID NO : 2, respectively, can be used to increase the thermotolerance or thermostability of known phospholipases not containing the respective aspartate residue. The standard methods for manipulation of nucleic acid sequences as set out above and known to the skilled person may be used to include the codon encoding the aspartate residue.

An example starting from the prior art sequence of PLC as published e.g. in Johansen, T., et al., Cloning and sequencing of the gene encoding the phosphatidylcholine-preferring phospholipase C of Bacillus cereus. Gene, 1988. 65: p. 293-304 or from Bacillus cereus strain ATCC 10987 or any other B. cereus strain publicly available is given below:

Upon cloning of the PLC gene by standard methods as described herein, e.g. in the TOPO®vector (see below), the Quik-Change® Site-Directed Mutagenesis Kit (Stratagene, DE) is used according to the instructions of the manufacturer (see also www.stratagene.com/manuals/200518.pdf) and e.g. the following primers:
FW: 5'- GGTACAAATCCAGAAXXXTGGATCCATGGAGCG - 3'
RV: 5' - CGCTCCATGGATCCAXXXTTCTGGATTTGTACC - 3'

Wherein XXX is a codon encoding aspartate (i.e. GAC or GAT; position 5 in SEQ ID NO:1 and position 174 in SEQ ID NO:2). In general, the mutagenic oligonucleotide primers for use in this protocol must be designed individually according to the desired mutation. The following preferred considerations should be made for designing mutagenic primers: Both of the mutagenic primers must contain the desired mutation and anneal to the same sequence on opposite strands of the plasmid. Primers should be between 25 and 45 bases in length, with a melting temperature (Tm) of ≥78°C. Primers longer than 45 bases may be used, but using longer primers increases the likelihood of secondary structure formation, which may affect the efficiency of the mutagenesis reaction. The desired mutation (deletion or insertion) should preferably be in the middle of the primer with ~10-15 bases of correct sequence on both sides. The primers optimally should have a minimum GC content of 40% and should terminate in one or more C or G bases.

As a further alternative, a phospholipase as described herein may be synthesized using standard oligopeptide synthesis methods as known in the art.

The following deposits have been made at the Deutsche Sammlung von Mikroorganismen und Zellkulturen GmbH (DSMZ), Mascheroder Weg 1b, D-38124 Braunschweig in accordance with the Budapest treaty on 18 November 2005 in the name of Süd-Chemie AG, Lenbachplatz 6, 80333 München:
1. pMSE3-516 in Bacillus subtilis. The reference number is "pMSE3-516"; the official deposit number is DSM 17731;
2. Bacillus cereus strain 516. The reference number is "SBUG516"; the official deposit number is DSM 17730.

Herein, the terms "B. cereus strain 516", "B. cereus 516" and "B. cereus SBUG516" or "SBUG516" are used interchangeably and refer to the same microorganism as deposited. The host organism, B. subtilis as used for pMSE3-516 is further described herein and in Wu, S.-C., Yeung, J. C., Duan, Y., Ye, R., Szarka, S. J., Habibi, H. R., Wong, S.-L.. Functional production and characterization of a fibrin-specific single-chain antibody fragment from Bacillus subtilis: Effects of molecular chaperons and a wall-bound protease on antibody fragment production, Appl. Environ. Microbiol., 2002, 68: p.3261-3269, regarding B. subtilis strain WB800. B. cereus and B. subtilis have the general features as described in R. E. Buchanan, N. E. Gibbons: Bergey's Manual of Determinative Bacteriology. Both organisms may be cultures as well known to the skilled person on LB agar plates or in LB (Luria-Bertani) medium as described in the Examples.

The invention is now described in more detail by way of the following non-limifing examples.

The figures show:

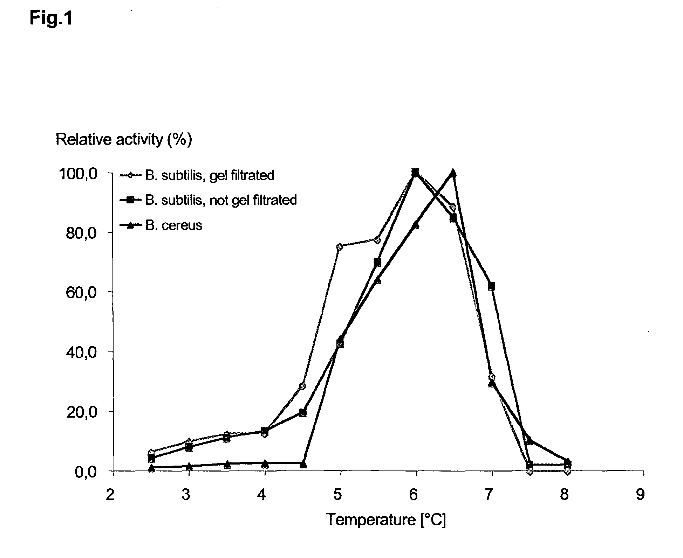

Fig. 1 shows the temperature profiles of the PLC as used in the present invention (comprising SEQ ID NO:1 and SEQ ID NO:2, respectively) in different enzyme preparations: from B. cereus 516 ("B.cereus"; from supernatant after ammonium sulphate precipitation and gel filtration as described below (4.)) and from expression in B. subtilis ("B. subtilis") using pMSE3-516 (with or without prior gel filtration, see below (4.));

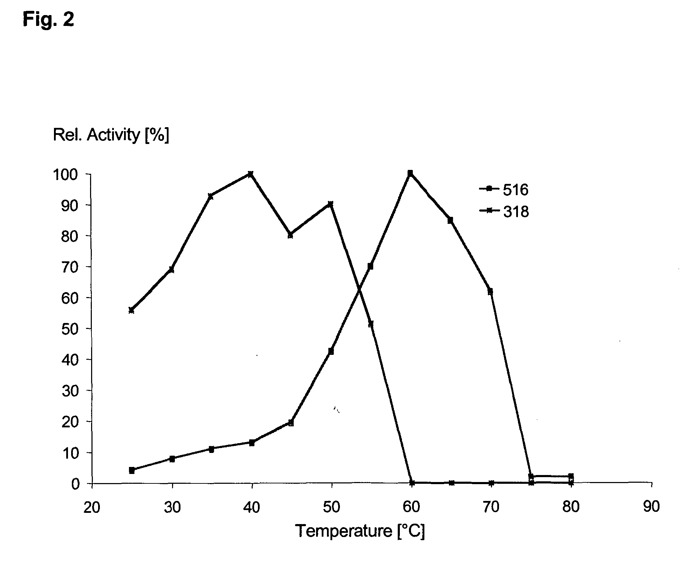

Fig. 2 shows a comparison of the temperature profiles of PC-PLCs from *B. cereus* strains 318 (from supernatant after ammonium sulphate precipitation and gel filtration as described below (4.); comparison) and 516 (produced in B. subtilis using pMSE3-516 as described below; according to the invention), respectively;

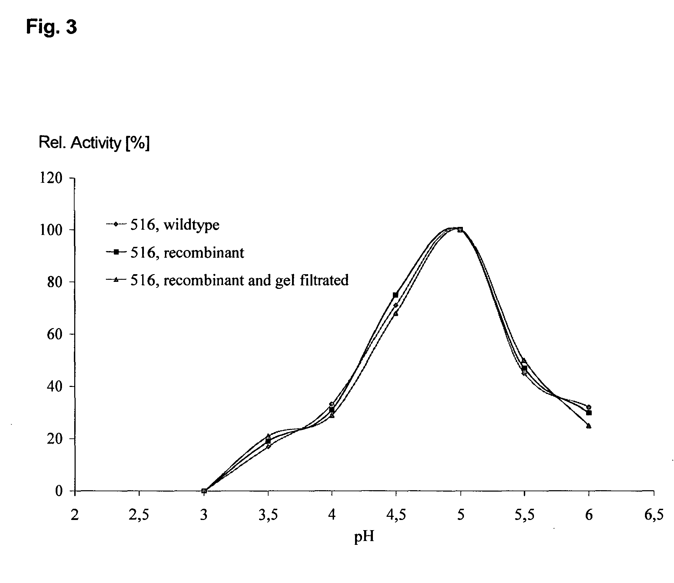

Fig. 3 shows pH profiles of the different PC-PLC preparations of the "wildtype" enzyme (from supernatant after ammonium sulphate precipitation and gel filtration as described below (4.)) and the "recombinant" enzyme from B. cereus 516 (produced in B. subtilis via pMSE3-516 as described below; gel filtration as indicated);

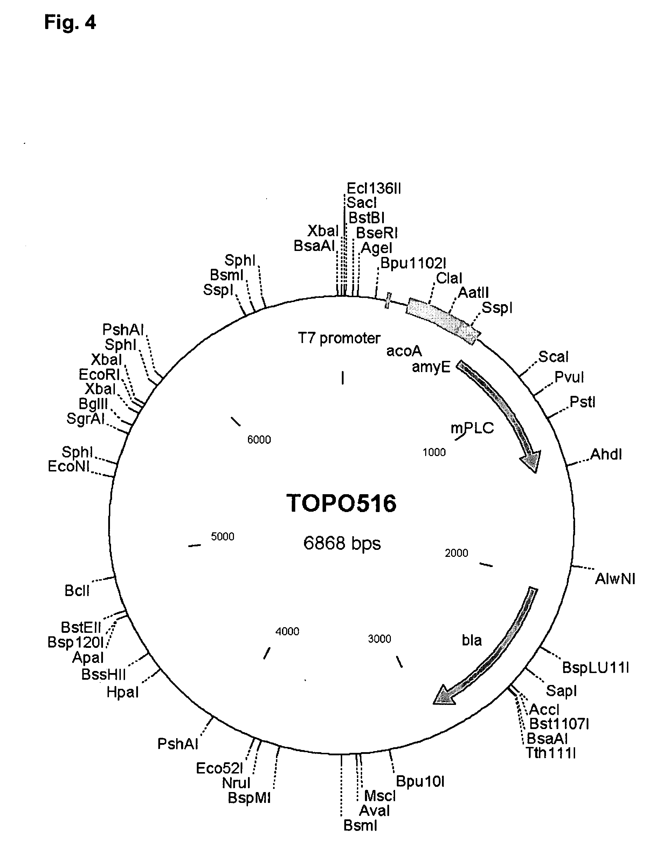

Fig. 4 shows a map of the TOPO 516 vector including the PLC from B. cereus 516; The features of the TOPO® vector (Invitrogene, DE) are summarized below and in www.invitrogene.com/contents/sfs/manuals/pettopo_man.pdf.

| **Feature** | **Benefit** |
|---|---|
| T7 promoter | Permits high-level, IPTG-inducible expression of your recombinant protein in E. coli strains expressing the T7 RNA polymerase. |
| T7 forward priming site lac operator (lacO) | Allows sequencing in the sense orientation. Binding site for lac repressor that serves to reduce basal expression of your recombinant protein. |
| Ribosome binding site | Optimally spaced from the TOPO® Cloning site for efficient translation of PCR product. |
| TOPO® Cloning site (directional) | Permits rapid cloning of your PCR product for expression in E. coli. |
| C-terminal V5 epitope tag (Gly-Lys-Pro-ile-Pro-Asn-Pro-Leu-Leu- Gly-Leu-Asp-Ser-Thr) | Allows detection of the fusion protein by the Anti-V5 Antibodies (Southern et al., 1991). |
| C-terminal 6xHis tag | Permits purification of recombinant fusion protein on metal-chelating resins (i.e. ProBond^{™} or Ni-NTA). |
| | In addition, allows detection of recombinant protein with the Anti-His(C-term) Antibodies (Lindner et al., 1997). |
| T7 Reverse priming site | Allows sequencing of the insert. |
| T7 transcription termination region | Sequence from T7 bacteriophage which permits efficient transcription termination. |
| bla promoter | Allows expression of the ampicillin resistance gene. |
| Ampicillin resistance gene (β-lactamase) | Allows selection of the plasmid in E. coli. |
| pBR322 origin of replication (ori) | Permits replication and maintenance in E. coli. |
| ROP ORF | Interacts with the pBR322 origin to facilitate lowcopy replication in E. coli. |
| lacl ORF | Encodes lac repressor which binds to the T7lac promoter to block basal transcription of the gene of interest and to the lacUV5 promoter in the host chromosome to repress transcription of T7 RNA polymerase. |

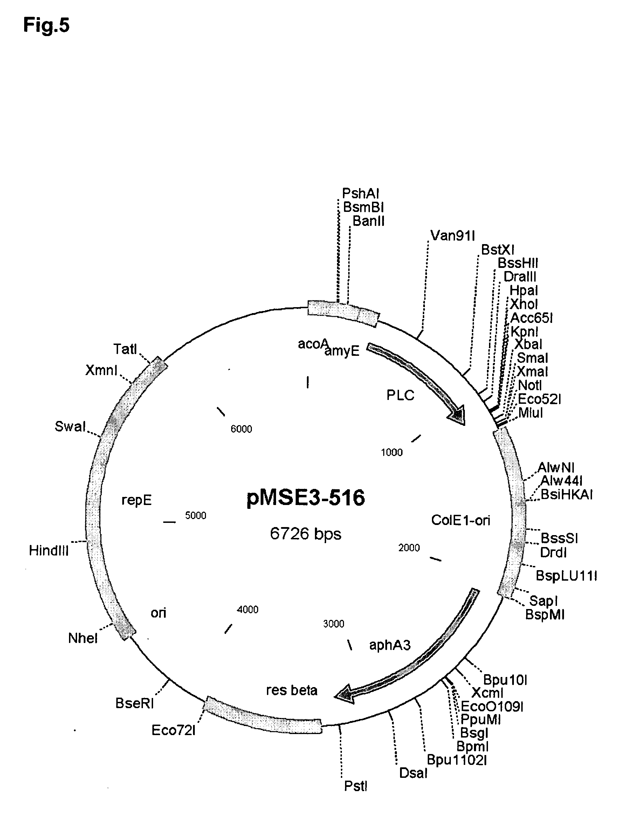

Fig. 5 shows a map of the pMSE3-516 vector including the PLC from B. cereus 516. The features of the vector are summarized below:

| | |
|---|---|
| location | Function |
| 1-88 bp | multiple cloning site |
| 106-978 bp | ColEl-ori (origin of replication for E. coli) |
| 1001-2083 bp | aphA3 (resistance against Kanamycin) |
| 2175-2778 bp | res beta (stabilises the plasmid) |
| 3292-3333 bp | ori (origin of replication for B. subtilis) |
| 3336-4826 bp | repE |

wherein the following abbreviations are used:
acoA: acetoin dehydrogenase promoter from B. subtilis
amyE: alpha-amylase-signal sequenz from B. subtilis
ColE1: replikation origin of E. coli plasmid
aphA3: kanamycine resistence from Enterococcus faecalis
res beta: resolvase gene from B. subtilis for stabilistation of the plasmid
repE: replication initiation protein from E. coli

Further information on the restriction enzymes and the respective abbreviations are to be found e.g. at www.neb.com/nebecomm/products/category1.asp?#2.

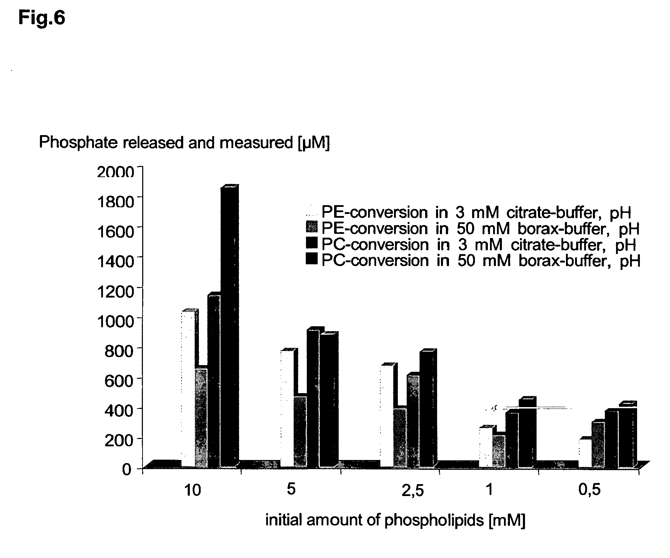

Fig. 6 shows the conversion of two different phospholipids (1,2-dimyristoyl phosphoryl choline (PC) and 1,2-dipalitoyl ethanolamine (PE)) in an oil-water two phase system by the PC-PLC according to the invention.

The attached Sequence Listing comprises SEQ ID NO:1 (1), SEQ ID NO:2 (2), SEQ ID NO:3 (3), SEQ ID NO:4 (4) and SEQ ID NO:5 (5) as outlined herein. B. cereus was obtained from environmental samples.

### 1. Materials and Methods

All chemicals and kits were obtained at the highest purity available from common commercial suppliers (Fluka, Aldrich, Signa, Promega, Roth, PeqLab, Merck, ABCR, QlAgen, Invitrogen, Amersham Biosciences, Genlal). B. cereus strains 318 and 516 were provided by Prof. Dr. Frieder Schauer, Greifswald University. The *p*-nitrophenylphosphorylcholine was synthesized as described before ( Durban, M. and U.T. Bornscheuer, An assay system for the detection of phospholipase C activity. Eur. J. Lipid Sci. Technol., 2003. 105: p. 633-637.). The plasmid pMSE3 and the acoAamyE sequence and *Bacillus subtilis* strains, e.g. Bacillus subtilis strains WB800, 168 or. 5061 a usable for pMSE3, were obtained from Prof. Dr. Thomas Schweder, Greifswald University (see also e.g. Wu, S.-C., Yeung, J. C., Duan, Y., Ye, R., Szarka, S. J., Habibi, H. R., Wong, S.-L.. Functional production and characterization of a fibrin-specific single-chain antibody fragment from Bacillus subtilis: Effects of molecular chaperons and a wall-bound protease on antibody fragment production, Appl. Environ. Microbiol., 2002, 68: p.3261-3269). Other B. subtilis strains and other bacterial expression vectors may be used.

### 2. Cloning of the plc gene

The DNA of B. cereus 516 was isolated with a commercially available DNA isolation kit (Fa. Gen-IAL GmbH, D-53840 Troisdorf; nach Angaben des Herstellers). The primers (FW-mPLC: 5'-TGG TCT GCT GAA GAT AAA CAT AAA GAA GG-3', PLC-RW-Xba: 5'-CCG TCT AGA TTA ACG ATC TCC GTA CG-3') were designed using the commonly known sequence of PC-PLC (Johansen, T., et al., Cloning and sequencing of the gene encoding the phosphatidylcholine-preferring phospholipase C of Bacillus cereus. Gene, 1988. 65: p. 293-304.). At the 3' end the TOPO recognition sequence and an *Xba*l site and at the 5' end an overhang for the following fusion PCR was created (Fus-acoA-amyE-mPLC (5'-GGC TGC GAG TGC TTG GTC TGC TGA AG-3') and PLC-RW-*Xba* (5'-CCG TCT AGA TTA ACG ATC TCC GTA CG-3')). The gene was amplified using a standard PCR protocol (Sambrook, J., Fritsch, E. F., Maniatis, T. (1989), Molecular Cloning: A Laboratory Manual, Cold Spring Harbour, NY). The acoAamyE sequence was amplified using primers, which make it possible to create an overhang for the following fusion PCR at the 3' end and an Xbal site at the 5' end (Xba-Topo (5'-CAC CTC TAG ATC AGT CAA ACG ATG-3') and Fus_mPLC_aco_amy_RW (5'-CTT CAG CAG ACC AAG CAC TCG CAG CC-3')). The two sequences were fused and cloned into a TOPO-vector (Invitrogen, Carlsbad, USA) to yield TOPO 516 (see Figure 4). After the transformation of the plasmid into *E. coli* XL 21 blue, the plasmid was purified and digested with Xbal. The 1100 kb fragment containing the fused acoAamyEmPLC sequence was ligated into a *Xba*l-cut pMSE3 to yield pMSE3-516 (see Figure 5). The plasmid was transformed into E. coli (DH5a) and sequenced. The plasmid was purified and transformed into *B. subtilis.* SEQ ID NO:4 shows the nucleotide sequence of the combined acoAamyEmPLC sequence as used in the cloning. The acoA promoter sequence extends to position 265. The ATG start codon is located at position 266 to 268 of SEQ ID NO:4. The coding sequence of amyE signal peptide from B. subtilis extends from position 266 to position 363. The coding sequence of PC-PLC from B.cereus extends from position 364 to position 1099 (positions 1100 to 1102 constitute the stop codon). SEQ ID NO:5 shows the respective amino acid sequence of the amyE signal sequence and the PC-PLC polypeptide.

### 3. p-Nitrophenylphosphorylcholine-(NPPC) Assay

10 µl of a 100 mM solution of NPPC in borax-HCl buffer (100 mM, pH 5-9) and 90 µl of an enzyme solution (lyophilized PLC re-dissolved in borax-HCl buffer (100 mM, pH 5-9)) are mixed in a well of a microtiterplate. *p*-nitrophenol released is then quantified by measurement of its absorbance at 410 nm during 30 minutes in 30 second steps in a microtiterplate reader (FLUOstar galaxy, BMG, Offenburg, Germany).

Calibration curves were created with varying concentrations of *p*-nitrophenol. At temperatures higher than 45°C 100 µl of a 100 mM solution of NPPC in Borax-HCl buffer (100 mM, pH 5-9) and 900 µl of an enzyme solution (lyophilized PLC re-dissolved in Borax-HCl buffer (100 mM, pH 5-9)) are mixed in an Eppendorff tube and incubated at different temperatures in a thermoshaker (Eppendorff). Every four minutes a sample of 100 µl was taken and *p*-nitrophenol released is then quantified by measurement of its absorbance at 410 nm in a microtiterplate reader (FLUOstar galaxy, BMG, Offenburg, Germany).

One unit of activity of the enzyme is generally defined as the amount of enzyme forming 1 micromol of *p*-nitrophenol.

### 4. Cultivation of B. subtilis and expression of the PC-PLC

For the cultivation of the B. subtilis with pMSE3-516, a 50 ml preculture was prepared with a 5 ml over night culture. Standard LB medium (see above) or an enriched "super broth" (SB) medium may be used. SB medium may be prepared by mixing 950 ml of solution 1 (32 g tryptone, 20 g yeast extract, 5 g NaCl, 5 ml 1N NaOH in 1 L water; autoclaved) and 50 ml solution 2 (12 g Na₂HPO₄, 6 g KH₂PO₄, 2 g NaCl and 6 g CaCl₂ in 100ml water, autoclaved). The above preculture was cultivated at 37 °C and 150 rpm until an OD600 of 0.85 was reached. This preculture was used to inoculate 4 L SB-media in a fermenter (New Brunswick Scientific, Edison, New Jersey, USA). Cultivation was then performed at 37°C and pH 6.5. The agitation and the air flow was controlled by oxygen content in the medium which was fixed at 20% dO₂. The expression of the PC-PLC was induced at an OD₆₀₀ of 3 by adding acetoin at a final concentration of 0.5 %. After 30 h the cells were separated from the medium by centrifugation (Multifuge 3 S-R, Kendro, Hanau, Germany; 15 min, 4°C, 4570 g) and the proteins in the supernatant (4I) were salted out with 90 % ammonium sulfate. The precipitated proteins were re-dissolved in Borax HCl buffer (100 mM, pH 7.5) and concentrated with centricons (Centricon Plus-20, 30000 Da, Millipore, Bedford, MA, USA). The PC-PLC was located in the retentate, while the flow-through showed no PC-PLC-activity. The flow-through was purified by gel filtration using a Sephacryl S-200 High Resolution column at a Äktaprime (Amersham Biosciences; flow: 2 ml/min, fraction size: 2 ml, 100 mM Borax-HCl, pH 7.5 as buffer). The fractions containing the PC-PLC were concentrated with centricons again to reduce the volume.

To compare the PC-PLC activities, B. cereus 516 (comprising PC-PLC with SEQ ID NO:2) and 318 (comprising PC-PLC identical to SEQ ID NO:2 with the exception of glutamate instead of aspartate at position 174 of the amino acid sequence) were cultivated under the same condition as described above until the highest PC-PLC activity in the supernatant was reached. The enzyme was purified as described above. All PC-PLC activities were determined with the NPPC assay as described above.

### 5. Gel electrophoresis and activity staining

Sodium dodecyl sulfate (SDS) and native polyacrylamide gels were prepared as described (S. Lange A. Musidlowska, C. Schmidt-Dannert, J. Schmitt, U.T. Bornscheuer, Cloning, Functional Expression, and Characterization of Recombinant Pig Liver Esterase, ChemBioChem 2001, 2: p. 576-582). For activity staining, the native gel was incubated in a mixture of freshly prepared solutions A and B (1:1) for 12 h (A: α-NPC (50 mg) dissolved in 10 ml borax-HCl buffer (100 mM, pH7.5); B: Fast Red TR salt (10 mg) dissolved in the same buffer). The gel was then washed with distilled water and also stained for protein detection with Coomassie Brilliant Blue R250.

The isolated gene from B. cereus 516 that was cloned into the plasmid pMSE3 showed 13 differences in nucleotide sequence compared to the published one (Johansen, T., et al., Cloning and sequencing of the gene encoding the phosphatidylcholine-preferring phospholipase C of Bacillus cereus. Gene, 1988. 65: p. 293-304.). These differences led to only one difference in the amino acid sequence. On position 174 there is an aspartate instead of glutamate.

### 6. Expression of the PC-PLC in B. subtilis and purification of the enzyme

The production of recombinant PC-PLC from B. cereus in B. subtilis is a very easy way to produce high amounts of active enzyme. The PC-PLC activity in the medium increased after induction with acetoin constantly up to 200 U per ml medium after 30 h. In contrast to the enzyme production in the "wildtype", there is no degradation of the enzyme after reaching the maximum.

The crude protein lyophilysate obtained after the ammonium sulfate precipitation showed an activity of 1576 U/mg. After the ultrafiltration, the activity increased up to 1983 U/mg but there are still many different proteins in the extract. With a gelfiltration with Sephacryl S-200 High resolution a good purification of the PC-PLC can be obtained. The PC-PLC showed an apparent molecular weight of 26 kDa.This purified enzyme showed an activity of 13192 U/mg and was used for further characterizations.

### 7. Characterization of the recombinant PC-PLC

For industrial applications of the enzyme it is very important to know, whether the recombinant expression influences the activity of the PC-PLC. As for the use of enzymes in industry it is important to produce high amount of enzyme with low costs it also has to be checked, if there is a difference in activity when using the crude enzyme isolated from the culture medium and the purified enzyme. Therefore it is important to check the PC-PLC activity in the crude preparation as well as in the purified one. As evident from Fig.1, the enzyme was most active at a low pH and shows activity up to 80°C with a maximum at about 65°C. Surprisingly, in a composition comprising an oil or fat the thermal stability of the enzyme was even higher and the activity range and maximum was shifted to higher temperatures.

As described above, the amino acid sequence of the PC-PLC of B. cereus strain 516 differs from the amino acid sequence of the PC-PLC of B. cereus strain 318 only by the presence of aspartate instead of glutamate at amino acid position 174. Importantly, the thermostability of the PLC from B. cereus strain 516 is however considerably higher than the thermostability of the PLC from B. cereus strain 318, see Figure 2. The same results were obtained with the PLCs obtained from the B. cereus strains (318 and 516) directly or after cloning the PLC genes in pMSE3 and expression in B. subtilis. Between the crude preparation and the purified enzyme there is also only a minor difference. In the pH profile no differences between the different preparations of the recombinant enzyme and the wildtype PC-PLC were determined (see Fig. 3).

### 8. Activity of the phospholipase according to the invention in an oil-water two phase system.

For this series of experiments a PC-PLC reaction and molybdate assay as follows was used:

500 µl solvent with a phospholipid concentration between 10 µM and 10 mM was mixed with 500 µl PC-PLC solution (205 U/ml or 730 U/ml) in Borax-HCl (100 mM, pH 7,5), 200 µl Borax-HCl and 25 µl alkaline phosphatase (60 U/ml). The samples were incubated for 12 to 18 h at 37°C and 300 rpm in an incubation shaker (Eppendorff, Hamburg, Germany).

To 150 µl of the aqueous phase, 75 µl HCl, 450 µl distilled water and 150 µl of a 2% ammonium molybdate solution in distilled water were added. The samples were shaken and incubated for 3 minutes at room temperature. The formed heteropolic acid was extracted with 600 µl butanol and the organic phase was washed twice with 375 µl distilled water. To the organic phase, a saturated SnC12 solution in 10 % HCl was added, shaken for 15 seconds and washed twice with 600 µl distilled water. 150 µl of the organic phase were taken and the blue colour was measured at 700 nm. For calibration, phosphate solutions of different concentrations were used and treated as described above. The overall principle of the method is shown below:

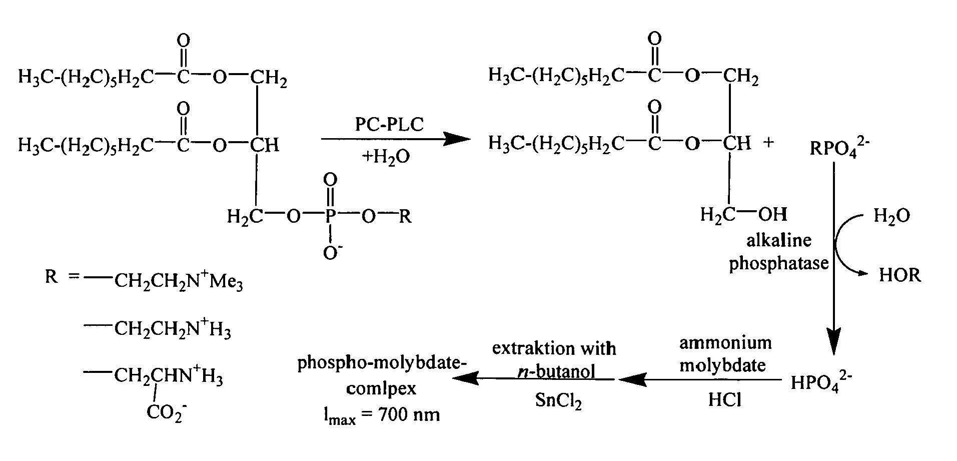

In the first step, the released phosphate is treated with alkaline phosphatase followed by conversion to the phosphomolybdate complex. This is then extracted with n-butanol and reduced by an acidic SnCl2 solution. The blue complex is then quantified by spectrophotometric measurement at 700 nm.

The above assay proved sensitive down to 1 ppm phosphate (5.2 micromole/l) and even less. Thus, rape seed oil (see above) was spiked with 10, 5, 2.5, 1 and 0.5 mM of two different phospholipids, 1,2-dimyristoyl phosphoryl choline (PC) and 1,2-dipalitoyl ethanolamine (PE), respectively, see Figure 6. 0.5 ml of the spiked oils were thoroughly mixed with 0.5 ml PLC solution (PLC from B. cereus 516 with SEQ ID NO:2; 730 U/ml in borax buffer or citrate buffer (see above) including 25 µl aqueous solution of alkaline phosphatase) and then incubated at 37 °C for 18 hours under agitation before analysis of the phosphate content as set out above. The results are shown in Fig. 6. The PC was converted best, followed by the PE.

### 9. Activity of the phospholipase according to the invention in the degumming of oil

100 g of a conventionally water-degummed rapeseed oil (0,78% FFA) were treated at 60°C with 2g of a 12.5% solution of citric acid in water for about 15 min. The acid treatment was followed by a neutralisation step to adjust the pH between 5 and 6 using NaOH ((2.5 g of 0.5 M NaOH). Then, the recombinant PLC expressed in B. subtilis using pMSE3-516 (289 U/mg; 34,6 mg/10ml buffer) was added in the amounts as evident from the Table below:

| **Rape seed oil [g]** | **Aqueous phase [ml]** | **Enzyme [ml]** | **Units of enzyme [U]** | **Temperature [°C]** |
|---|---|---|---|---|
| 100 | 15 | 0 | 0 U | 60 °C |
| 100 | 15 | 0.1 | 100 U | 60 °C |
| 100 | 15 | 0.5 | 500 U | 60 °C |
| 100 | 15 | 1.0 | 1000 U | 60 °C |

The amount of the aqueous phase (overall in the mixture) is also indicated and was adjusted to 15 ml using the respective enzyme buffer. Both a 50 mM borax buffer, pH 7,5 and a 3 mM citrate buffer, pH 5,5 were used. The pH value of the mixture was about 5.7 using citrate buffer. The incubation was carried out at 60 °C for 360 min. Subsequently, the oil phase was separated by centifugation and the phosphorous content (P-content) was determined. The oil had a phosphorous content of less than 5 ppm, even if only 100U of enzyme were used. The phosphorous content in the experiment using the borax buffer was even lower than in the experiment with citrate buffer.

The same low phosphorous contents were obtained when a water degummed oil with a phosphorous content of about 45 ppm was directly subjected to the enzymatic degumming as set out above.

## Claims

1. Use of a phospholipase comprising SEQ ID NO: 1:
TNPEDWIHG
or having at least 75% sequence identity to SEQ ID NO : 1 and comprising aspartate at position 5 of SEQ ID NO: 1
in a process for the cleavage and/or removal of at least one substrate of the phospholipase from a composition comprising the substrate of the phospholipase and at least one oil or fat.

2. Use according to claim 1, wherein the substrate of the phospholipase comprises or consists of at least one phospholipid, preferably at least one non-hydratable phospholipid.

3. Use according to any one of the preceding claims, wherein the process comprises the following steps:
a) providing the phospholipase,
b) providing a composition comprising at least one substrate for the phospholipase and at least one oil or fat;
c) contacting the phospholipase with the composition to obtain a mixture comprising the phospholipase and its substrate (at at least one oil or fat);
d) incubating the mixture, preferably at a temperature above 20 °C.

4. Use according to any one of the preceding claims, wherein the phospholipase is a phospholipase C (PLC).

5. Use according to any one of the preceding claims, wherein in the incubation, the phospholipase is allowed to cleave the substrate, and, preferably, the cleaved substrate is then removed from the oil or fat.

6. Use according to any one of the preceding claims, wherein the process is a process for the treatment or degumming of an oil or fat, in particular a plant oil, an animal oil, an algae or a fish oil or a fat.

7. Use according to any one of the preceding claims, wherein the plant oil is selected from the group consisting of a soybean oil, a rapeseed oil, a corn oil, an oil from a palm kernel, a canola oil, a sunflower oil, a sesame oil and/or a peanut oil.

8. Use according to any one of the preceding claims, wherein the composition comprises an oil phase and a water phase, preferably in the form of an emulsion of the oil phase and the water phase.

9. Use according to claim 4, wherein the incubation is carried out at a temperature of 37 °C or above, preferably 50 °C or above, preferably 55 °C or above, preferably 60 °C or above, more preferred 65 °C or above, further preferred 75 °C or above, in particular 80 °C or above or even 85 °C or above.

10. Use according to the preceding claims, wherein the polypeptide is provided in the form of an aqueous solution or immobilised on a carrier or carrier particles.

11. Use according to the preceding claims, wherein the substrate is provided in the form of an an oil or fat comprising the substrate, in particular an oil or fat comprising phospholipids.

12. Use according to the preceding claims, wherein the substrate is a phospholipid in an edible oil or fat.

13. Use according to the preceding claims, wherein the phospholipase is selected from the polypeptides comprising SEQ ID NO: 2 or having at least 75% sequence identity to SEQ ID NO: 2 over a region of at least about 100 residues, wherein the sequence identities are determined by analysis with a sequence comparison algorithm or by a visual inspection, the polypeptide comprising the amino acid aspartate at position 174 of SEQ ID NO: 2.

14. Use according to the preceding claims, wherein the phospholipase is selected from the polypeptides encoded by a nucleic acid according to SEQ ID NO : 3 or a sequence having at least 75% sequence identity to SEQ ID NO : 3 over a region of at least about 100 residues, wherein the sequence identities are determined by analysis with a sequence comparison algorithm or by a visual inspection, or encoded by a sequence hybridizing to SEQ ID NO : 3 under stringent conditions, the stringent conditions preferably including a wash step comprising a wash in 0. 2X SSC at a temperature of about 65 °C for about 15 minutes.

15. Use according to the preceding claims, wherein the sequence identity is at least about 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99%, or more, or is 100% sequence identity.

16. Use according to the preceding claims, wherein the sequence identity is over a region of at least about 10,15, 20,25, 30,35, 40,45, 50,75, 100, 150,200, 250,300, 350,400, 450,500, 550,600, 650,700, 800, 900, 1000 or the full length of SEQ ID 3 or over a region of at least about 10,15, 20,25, 30,35, 40,45, 50,75, 100, 150,200, 250, or the full length of SEQ ID NO 2, respectively.

17. Use according to the preceding claims, wherein the phospholipase activity comprises catalyzing hydrolysis of a phosphoric acid diester linkage, preferably a phospholipase C (PLC) acivity, more preferably a PC-PLC activity.

18. Use according to the preceding claims wherein the phospholipase activity comprises catalyzing hydrolysis of an ester linkage in a phospholipid in a vegetable oil, in particular an oilseed phospholipid.

19. Use according to claim 18, wherein the vegetable oil phospholipid is derived from a plant oil, a high phosphorous oil, a soy oil, a canola oil, a palm oil, a cottonseed oil, a corn oil, a palm kernel-derived phospholipid, a coconut oil, a peanut oil, a sesame oil, a fish oil, an algae phospholipid, a sunflower oil, an essential oil, a fruit seed oil, a grapeseed phospholipid, an apricot phospholipid, or a borage phospholipid.

20. Use according to the preceding claims, wherein the phospholipase (activity) is thermotolerant or thermostable.

21. Use according to the preceding claims, wherein the phospholipase retains a phospholipase activity under conditions comprising a temperature range of between about 37 to about 95 °C, between about 55 to about 85 °C, between about 60 to about 95 °C, between about 65 C to about 75 °C, or between about 90 C to about 95 °C.

22. Use according to the preceding claims, wherein the phospholipase has a maximum of its phospholipase activity at a temperature above 50 °C, preferably above 55°C, more preferably above 60 °C.

23. Use according to the preceding claims, wherein the phospholipase activity comprises a specific activity at about 60 °C of at least about 100, preferably at least about 200, more preferably at least about 500 units per milligram of protein, more preferably at least about 1000 units per milligram of protein, more preferably at least about 5000 units per milligram of protein, more preferably at least about 10000 units per milligram of protein.

24. Use according to the preceding claims, wherein, the phospholipase has a phospholipase activity at a temperature above 70 °C which is at least 50% of its maximum phospholipase activity, i.e. the phospholipase activity at the temperature optimum.

25. Use according to the preceding claims, wherein, the phospholipase has a maximum of its phospholipase activity at a pH in the range of 4 to 6, preferably in the range of 4,5 to 6.

26. Use according to the preceding claims, wherein, the phospholipase has a heterologous signal sequence.

27. Use according to the preceding claims, wherein, the phospholipase is immobilized on a solid carrier, preferably a cell, a metal, a resin, a polymer, a ceramic, a glass, a microelectrode, a graphitic particle, a bead, a gel, a plate, an array or a capillary tube, in particular a silicate or layer silicate carrier, preferably natural or synthetic smectites; acid-activated smectites, particularly bentonites; alkali-activated smectites, particularly bentonites; organically modified smectites, particularly bentonites; or hydrotalcites.

28. Use according to the preceding claims, wherein, the phospholipase is bound to a filter and a phospholipid-containing fat or oil is passed through the filter, or wherein the phospholipase is added to a composition comprising the phospholipid-containing fat or oil and then the composition is passed through a filter.

29. A method of increasing thermotolerance or thermostability of a phospholipase polypeptide, the method comprising the steps of:
(a) providing the nucleic acid sequence encoding the phospholipase polypeptide;
(b) identifying the part of the nucleic acid sequence encoding SEQ ID 4: TNPEXWIHG, wherein X is an amino acid other than aspartate;
(c) modifying one or more nucleotides in the codon encoding amino acid X so that the codon for the amino acid aspartate results.


SEQUENCE LISTING

<110>  Süd-Chemie AG

<120>  Use of a thermostable phospholipase in the degumming of an oil or
        fat, and a method for obtaining a thermostable phospholipase

<130>  4465-X-20.431

<160>  5

<170>  PatentIn version 3.3

<210>  1
<211>  9
<212>  PRT
<213>  Bacillus cereus

<400>  1

Thr Asn Pro Glu Asp Trp Ile His Gly
1               5

<210>  2
<211>  245
<212>  PRT
<213>  Bacillus cereus

<400>  2

Trp Ser Ala Glu Asp Lys His Lys Glu Gly Val Asn Ser His Leu Trp
1               5                   10                  15

Ile Val Asn Arg Ala Ile Asp Ile Met Ser Arg Asn Thr Thr Leu Val
            20                  25                  30

Lys Gln Asp Arg Val Ala Gln Leu Asn Glu Trp Arg Thr Glu Leu Glu
        35                  40                  45

Asn Gly Ile Tyr Ala Ala Asp Tyr Glu Asn Pro Tyr Tyr Asp Asn Ser
    50                  55                  60

Thr Phe Ala Ser His Phe Tyr Asp Pro Asp Asn Gly Lys Thr Tyr Ile
65                  70                  75                  80

Pro Phe Ala Lys Gln Ala Lys Glu Thr Gly Ala Lys Tyr Phe Lys Leu
                85                  90                  95

Ala Gly Glu Ser Tyr Lys Asn Lys Asp Met Lys Gln Ala Phe Phe Tyr
            100                 105                 110

Leu Gly Leu Ser Leu His Tyr Leu Gly Asp Val Asn Gln Pro Met His
        115                 120                 125

Ala Ala Asn Phe Thr Asn Leu Ser Tyr Pro Gln Gly Phe His Ser Lys
    130                 135                 140

Tyr Glu Asn Phe Val Asp Thr Ile Lys Asp Asn Tyr Lys Val Thr Asp

145                 150                 155                 160

Gly Asn Gly Tyr Trp Asn Trp Lys Gly Thr Asn Pro Glu Asp Trp Ile
                165                 170                 175

His Gly Ala Ala Val Val Ala Lys Gln Asp Tyr Ser Gly Ile Val Asn
            180                 185                 190

Asp Asn Thr Lys Asp Trp Phe Val Lys Ala Ala Val Ser Gln Glu Tyr
        195                 200                 205

Ala Asp Lys Trp Arg Ala Glu Val Thr Pro Met Thr Gly Lys Arg Leu
    210                 215                 220

Met Asp Ala Gln Arg Val Thr Ala Gly Tyr Ile Gln Leu Trp Phe Asp
225                 230                 235                 240

Thr Tyr Gly Asp Arg
                245

<210>  3
<211>  735
<212>  DNA
<213>  Bacillus cereus

<400>  3
tggtctgctg aagataaaca taaagaaggc gtaaattctc atttatggat tgtaaaccgt      60

gcgattgata ttatgtctcg caatacaaca cttgtaaaac aagatcgagt tgcacaatta     120

aatgaatggc gtacagagtt agagaacggt atttatgctg ctgactatga aaatccttat     180

tatgataata gcacatttgc ttcacatttc tatgatccag acaatggaaa aacatatatt     240

ccatttgcaa agcaggcaaa agaaactgga gctaaatatt ttaaattagc gggtgaatca     300

tacaaaaata aagatatgaa acaagcattc ttctatttag ggttatctct tcattattta     360

ggagatgtaa atcaaccgat gcatgcggca aactttacaa atctttcgta tccacaagga     420

ttccattcta aatatgaaaa ctttgtagat acgataaaag ataattataa agtaacggat     480

ggaaatggat attggaattg gaaaggtaca aatccagaag attggatcca tggagcggca     540

gtagtggcga aacaagatta ctctggaatt gtaaatgata atacgaaaga ttggttcgta     600

aaagcagctg tgtcacaaga atatgcagat aaatggcgtg ctgaagttac accgatgaca     660

ggtaagcgat taatggatgc acaacgtgtt actgctggat acattcagct ttggtttgat     720

acgtacggag atcgt                                                      735

<210>  4
<211>  1102
<212>  DNA
<213>  Bacillus cereus

<220>
<221>  misc_feature

<222>   (1)..(265)
<223>   acoA promoter sequence from B. subtilis

<220>
<221>   misc_feature
<222>   (266)..(268)
<223>   atg start codon

<220>
<221>   misc_feature
<222>   (266)..(363)
<223>   coding sequence of amyE signal peptide from B. subtilis

<220>
<221>   misc_feature
<222>   (364)..(1099)
<223>   coding sequence of PC-PLC from B.cereus

<400>   4
tcagtcaaac gatgcagagg aactaggaag ttaaaaagat ttccaaggaa ataaatacgt      60

cgatcattgt caaaggccgg gtgatatccg gtcttttttt tgcatgctgt aaaacgagac     120

aaatgaatca gtttgagaca aaacgagaca cacgtctcaa actgtctcca aagtgaagat     180

gagaagactg attttacggg ctcaaaagac tggcacactt cttgcattta taatggtgaa     240

ccctaaatag aaggaggcgc acaaaatgtt tgcaaaacga ttcaaaacct ctttactgcc     300

gttattcgct ggatttttat tgctgtttca tttggttctg gcaggaccgg cggctgcgag     360

tgcttggtct gctgaagata aacataaaga aggcgtaaat tctcatttat ggattgtaaa     420

ccgtgcgatt gatattatgt ctcgcaatac aacacttgta aaacaagatc gagttgcaca     480

attaaatgaa tggcgtacag agttagagaa cggtatttat gctgctgact atgaaaatcc     540

ttattatgat aatagcacat ttgcttcaca tttctatgat ccagacaatg gaaaaacata     600

tattccattt gcaaagcagg caaaagaaac tggagctaaa tattttaaat tagcgggtga     660

atcatacaaa aataaagata tgaaacaagc attcttctat ttagggttat ctcttcatta     720

tttaggagat gtaaatcaac cgatgcatgc ggcaaacttt acaaatcttt cgtatccaca     780

aggattccat tctaaatatg aaaactttgt agatacgata aaagataatt ataaagtaac     840

ggatggaaat ggatattgga attggaaagg tacaaatcca gaagattgga tccatggagc     900

ggcagtagtg gcgaaacaag attactctgg aattgtaaat gataatacga aagattggtt     960

cgtaaaagca gctgtgtcac aagaatatgc agataaatgg cgtgctgaag ttacaccgat    1020

gacaggtaag cgattaatgg atgcacaacg tgttactgct ggatacattc agctttggtt    1080

tgatacgtac ggagatcgtt aa                                             1102

<210>   5
<211>   278
<212>   PRT
<213>   Bacillus cereus

<220>
<221>   misc_feature
<222>   (1)..(33)
<223>   amyE signal peptide sequence from B. subtilis

<220>
<221>  misc_feature
<222>  (34)..(278)
<223>  PC-PLC coding sequence from B. cereus

<400>  5

Met Phe Ala Lys Arg Phe Lys Thr Ser Leu Leu Pro Leu Phe Ala Gly
1               5                   10                  15

Phe Leu Leu Leu Phe His Leu Val Leu Ala Gly Pro Ala Ala Ala Ser
            20                  25                  30

Ala Trp Ser Ala Glu Asp Lys His Lys Glu Gly Val Asn Ser His Leu
        35                  40                  45

Trp Ile Val Asn Arg Ala Ile Asp Ile Met Ser Arg Asn Thr Thr Leu
    50                  55                  60

Val Lys Gln Asp Arg Val Ala Gln Leu Asn Glu Trp Arg Thr Glu Leu
65                  70                  75                  80

Glu Asn Gly Ile Tyr Ala Ala Asp Tyr Glu Asn Pro Tyr Tyr Asp Asn
                85                  90                  95

Ser Thr Phe Ala Ser His Phe Tyr Asp Pro Asp Asn Gly Lys Thr Tyr
            100                 105                 110

Ile Pro Phe Ala Lys Gln Ala Lys Glu Thr Gly Ala Lys Tyr Phe Lys
        115                 120                 125

Leu Ala Gly Glu Ser Tyr Lys Asn Lys Asp Met Lys Gln Ala Phe Phe
    130                 135                 140

Tyr Leu Gly Leu Ser Leu His Tyr Leu Gly Asp Val Asn Gln Pro Met
145                 150                 155                 160

His Ala Ala Asn Phe Thr Asn Leu Ser Tyr Pro Gln Gly Phe His Ser
                165                 170                 175

Lys Tyr Glu Asn Phe Val Asp Thr Ile Lys Asp Asn Tyr Lys Val Thr
            180                 185                 190

Asp Gly Asn Gly Tyr Trp Asn Trp Lys Gly Thr Asn Pro Glu Asp Trp
        195                 200                 205

Ile His Gly Ala Ala Val Val Ala Lys Gln Asp Tyr Ser Gly Ile Val
    210                 215                 220

Asn Asp Asn Thr Lys Asp Trp Phe Val Lys Ala Ala Val Ser Gln Glu
225                 230                 235                 240

Tyr Ala Asp Lys Trp Arg Ala Glu Val Thr Pro Met Thr Gly Lys Arg
                245                 250                 255

Leu Met Asp Ala Gln Arg Val Thr Ala Gly Tyr Ile Gln Leu Trp Phe
            260                 265                 270

Asp Thr Tyr Gly Asp Arg
        275